(19) Europäisches Patentamt
European Patent Office
Office européen des brevets

(11) EP 1 966 259 B1

(12) FASCICULE DE BREVET EUROPEEN

(45) Date de publication et mention de la délivrance du brevet:
**18.04.2012 Bulletin 2012/16**

(21) Numéro de dépôt: **06841374.9**

(22) Date de dépôt: **14.12.2006**

(51) Int Cl.:
***C08F 220/38*** $^{(2006.01)}$ ***C08F 220/60*** $^{(2006.01)}$
***C08F 246/00*** $^{(2006.01)}$ ***C08F 8/34*** $^{(2006.01)}$
***A61K 8/81*** $^{(2006.01)}$ *C08F 220/56* $^{(2006.01)}$
*A61Q 5/02* $^{(2006.01)}$ *A61Q 5/12* $^{(2006.01)}$

(86) Numéro de dépôt international:
**PCT/EP2006/069730**

(87) Numéro de publication internationale:
**WO 2007/068744 (21.06.2007 Gazette 2007/25)**

# (54) COPOLYMERE COMPRENANT DES UNITES ZWITTERIONIQUES ET D'AUTRES UNITES, COMPOSITION COMPRENANT LE COPOLYMERE, ET UTILISATION

COPOLYMER MIT ZWITTERIONISCHEN EINHEITEN UND ANDEREN EINHEITEN, DAS COPOLYMER UMFASSENDE ZUSAMMENSETZUNG UND VERWENDUNG

COPOLYMER CONTAINING ZWITTERIONIC UNITS AND OTHER UNITS, COMPOSITION COMPRISING THE COPOLYMER, AND USE

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **14.12.2005 FR 0512670**

(43) Date de publication de la demande:
**10.09.2008 Bulletin 2008/37**

(73) Titulaire: **Rhodia Opérations**
**93306 Aubervilliers (FR)**

(72) Inventeurs:
- **BALASTRE, Marc**
  **F-75020 Paris (FR)**
- **CROOKS, Regan**
  **London, SW008RP (GB)**
- **VUONG, Chi-Thanh**
  **F-77185 Lognes (FR)**

(56) Documents cités:
**US-B1- 6 346 588**

- **WEN-FU L ET AL: "Poly(sulfobetaine)s and corresponding cationic polymers: 5. Synthesis and dilute aqueous solution properties of poly (sulfobetaine)s derived from acrylamide-maleic anhydride copolymer" POLYMER, ELSEVIER SCIENCE PUBLISHERS B.V, GB, vol. 37, no. 19, septembre 1996 (1996-09), pages 4389-4395, XP004069434 ISSN: 0032-3861**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 966 259 B1

## Description

La présente invention a pour objet un nouveau copolymère comprenant des unités zwitterioniques et d'autres unités, une nouvelle composition comprenant le copolymère, et l'utilisation du copolymère ou des compositions pour le traitement ou la modification de surfaces. Le copolymère, la composition, et l'utilisation présentent notamment un intérêt particulier dans le domaine de la cosmétique, notamment pour la réalisation de shampooings, après-shampooings ou gels douches, pour le conditionnement de la peau et/ou des cheveux. Ils présentent également un intérêt dans le domaine de la détergence, notamment pour les soins ménagers, industriels ou institutionnels.

Les polymères, homopolymères ou copolymères, comprenant des unités zwitterioniques sont connus. On connaît des compostions de tels polymères des utilisations dans différents domaines de l'industrie.

On connaît par exemple des copolymères comprenant et des unités à groupes carboxybétaïnes ou à groupes sulfobétaines.

Le document US4075131 décrit des polymères et leur introduction dans des shampooings conditionneurs. Des polymères décrits sont des homopolymères comprenant des unités zwitterioniques dérivant de monomères de la famille des carboxybétaïnes. Il s'agit d'homopolymères de carboxybétaïnes.

Le document US6403073 décrit des compositions cosmétiques destinées à être appliquées sur les cheveux, telles que des shampooings, comprenant un polyorganosiloxane (silicone), et un polymère polyampholythe insoluble. Le document décrit notamment des homopolymères comprenant des unités zwitterioniques bétaïnes. Il n'est pas décrit de comonomère pouvant être utilisé en copolymérisation avec les monomères bétaïnes.

Le document FR2742657 décrit des compositions cométiques destinées à être appliquées sur les cheveux, telles que des shampooings, comprenant un polymère cationique de densité de charge inférieure ou égale à 4 meq/g, et un polymère polyampholythe insoluble, identique à celui du document US4075131.

Le document US4534892 décrit des compositions telles que des shampooings comprenant un copolymère comprenant des unités bétaïne, des unités réticulantes, et éventuellement d'autres unités. Des monomères sulfobétaïne sont cités comme possibles comonomères bétaïne. Les exemples divulguent des copolymères comprenant des unités cationiques dérivées de QDM (2-methacryloxyethyltrimethylammonium contre-ion ethosulfate), et d'acide acrylique ou AMPS (monomères anioniques ou potentiellement anioniques) et d'un monomère réticulant.

Le document EP112592 décrit des polymères qui peuvent comprendre des unités bétaïne, et leur utilisation dans des compositions détergentes (lessives). En particulier le document décrit la famille des polyacrylates, polyacrylamides, comprenant des unités bétaïne, de formule IV page 16. On note qu'il s'agit d'homopolymères comprenant des unités bétaïne.

Le document FR2519863 décrit des compositions comprenant un composé cationique (tensioactif cationique ou polymère cationique) et un polymère bétaïnisé. Le polymère bétaïnisé comprend des unités bétaïne de type carboxybétaïne. Il est obtenu par réaction post polymérisation, par réaction d'un composé de formule $XCH_2COO^-$ sur un polymère comprenant des motifs portant un groupe amine tertiaire (potentiellement cationique). Un exemple est le polymère Amersette (Amerchol) ou le polymère Amphoset (Mitsubishi pétrochem.).

Le document US4994088 décrit des compositions pour cheveux, de type laque ou plus généralement des produits de mise en forme des cheveux ("styling"). Les compositions comprennent un polymère à unités bétaïnes. Dans les exemples 5 et 6, des homopolymères de sulfobétaïnes sont décrits.

Le document US4607076 décrit des copolymères comprenant des unités sulfobétaïne, par exemple des unités dérivant de SPE, et des unités vinyl-pyrrolidone. Les copolymères sont utilisés comme viscosants dans des compositions salines.

Le document EP532967 décrit des compositions cosmétiques, par exemple des shampooings, comprenant des copolymères de formule (1) comprenant des unités bétaïne (indice n), et des unités hydrophobes (indice m). Les unités bétaïne sont de carboxybétaïnes.

Le document WO 2004/083354 décrit des compositions pour le nettoyage de surfaces dures comprenant des polymères à unités bétaïne. Le document suggère des copolymères comprenant, en plus des unités bétaïne, des unités anioniques.

Le document WO 00/01746 décrit des copolymères à base d'acrylamide et de sulfobétaïnes ou de phosphobétaïnes. Il est indiqué dans ce document que ces copolymères sont efficaces comme agents viscosants, et comme agents modificateurs des surfaces de particules en suspension, dans le domaine de l'industrie pétrolière.

Le document US5026490 décrit d'autres copolymères comprenant des unités sulfobétaïne, et leur utilisation comme agents défloculants de boues de forage dans l'industrie pétrolière. Le document US6346588 décrit d'autres copolymères comprenant des unités sulfobétaïne, dont la formulation dans un fluide de forage est facilitée. Le document US4607076 décrit d'autres copolymères comprenant des unités sulfobétaïne, et leur utilisation dans l'industrie pétrolière comme agents viscosants en présence de saumure.

Par ailleurs, il est connu que la formation de coacervats est favorable au traitement ou à la modification de surfaces, notamment pour le conditionnement des cheveux et/ou de la peau. Ainsi on connaît l'utilisation de dérivés

cationiques de polysaccharides dans des shampooings comprenant des tensioactifs anioniques, de préférence en association avec des agents de conditionnement tels que de polyorganosiloxanes. Il a été enseigné que la formation de coacervats favorise le conditionnement. Les domaines de formulations où il est possible d'observer une formation de coacervat sont généralement limités, et dépendent généralement de la nature et des quantités des tensioactifs et des polymères mis en couve. Il existe un besoin pour des nouveaux polymères pouvant notamment participer à la formation de coacervats dans des domaines de formulations modifiés ou étendus.

Il existe par ailleurs dans l'industrie un besoin constant pour de nouveaux polymères pouvant apporter dans des compositions de nouvelles propriétés ou améliorer des propriétés.

L'invention répond à au moins un des besoins mentionnés ci-dessus en proposant un copolymère tel que défini dans la revendication 1.

L'invention concerne également des compositions comprenant le copolymère. Selon un mode préférentiel, la composition comprend des coacervats ou est susceptible de former des coacervats.

L'invention concerne également des coacervats comprenant le copolymère.

L'invention concerne également l'utilisation du copolymère dans des compositions.

L'invention concerne également un procédé de traitement ou de modification d'une surface, comprenant les étapes suivantes:

- appliquer sur la surface une composition comprenant le copolymère, et
- éventuellement, éliminer le vecteur ou diluer la composition ou modifier le pH.

L'invention concerne également la surface ainsi traitée ou modifiée.

L'invention concerne également un substrat dont la surface comprend le copolymère, par exemple sous forme de coacervats.

Le copolymère peut se déposer ou favoriser le dépôt d'un agent pour le traitement ou la modification de surface. En particulier, dans une composition cosmétique, par exemple dans des shampooings, après-shampooings ou gels douche, il peut procurer un conditionnement modifié ou amélioré des cheveux et/ou de la peau. Il peut favoriser le dépôt d'agents conditionneurs tels que des polyorganosiloxanes. La composition cosmétique peut notamment nettoyer et conditionner en procurant un meilleur peignage sur les cheveux mouillés. Pour pouvoir nettoyer, de fortes quantités de tensioactifs anioniques, non ioniques et/ou amphotères sont généralement utilisées dans la composition. Le conditionnement est obtenu par l'introduction du copolymère qui forme un revêtement sur les cheveux. Des huiles siliconées peuvent également être ajoutées. Il est connu que le dépôt de polymères et d'actifs (huiles silicones...) présents dans un shampooing peut être amélioré par la formation de coacervats entre un polymère cationique et des tensioactifs anioniques. Le copolymère selon l'invention peut notamment permettre la formation de coacervats dans la composition en présence de tensioactifs. On a trouvé notamment que cette formation peut être favorisée en jouant sur des paramètres tels que la nature et la composition du copolymère et des ingrédients présents dans la composition. La composition du copolymère et sa quantité peuvent être optimisée pour obtenir une composition qui est transparente et précipite (avec formation de coacervats par exemple) à la dilution. Les copolymères selon l'invention sont grandement modulables. Ils peuvent notamment comprendre des unités non ioniques qui peuvent augmenter leur faculté à être formulés dans différentes formulations et/ou pour différentes formulations. Leur modularité permet de les formuler dans différents environnements, elle permet aussi de régler les propriétés et performances des compositions où ils sont introduits.

Définitions

Dans la présente demande, on désigne par copolymère tout polymère comprenant au moins deux types d'unités. Le terme copolymère comprend les copolymère binaires, ne comprenant que deux types d'unités, les copolymères comprenant trois types d'unités (terpolymères) etc...

Dans la présente demande, on désigne par unité dérivant d'un monomère, pour les unités différentes des unités A, une unité qui peut être obtenue directement à partir dudit monomère par polymérisation. Ainsi, par exemple, une unité dérivant d'un ester d'acide acrylique ou méthacrylique ne couvre pas une unité de formule $-CH_2-CH(COOH)-$, $-CH_2-C(CH_3)(COOH)-$, $-CH_2-CH(OH)-$, respectivement, obtenue par exemple en polymérisant un ester d'acide acrylique ou méthacrylique, ou de l'acétate de vinyle, respectivement, puis en hydrolysant. Une unité dérivant d'acide acrylique ou méthacrylique couvre par exemple une unité obtenue en polymérisant un monomère (par exemple un ester d'acide acrylique ou méthacrylique), puis en faisant réagir (par exemple par hydrolyse) le polymère obtenu de manière à obtenir des unités de formule $-CH_2-CH(COOH)-$, ou $-CH_2-C(CH_3)(COOH)-$. Une unité dérivant d'un alcool vinylique couvre par exemple une unité obtenue en polymérisant un monomère (par exemple un ester vinylique), puis en faisant réagir (par exemple par hydrolyse) le polymère obtenu de manière à obtenir des unités de formule $-CH_2-CH(OH)-$.

Dans la présente demande, le terme «hydrophobe» est utilisé dans son sens usuel de «qui n'a pas d'affinité pour l'eau»; cela signifie que le polymère organique dont il est constitué, pris seul (de même composition et de même

masse molaire), formerait une solution macroscopique diphasique dans de l'eau distillée à 25°C, à une concentration supérieure à 1% en poids.

Dans la présente demande, les termes «hydrophile», "hydrosoluble" et "hydrodispersable" sont également utilisés dans leur sens usuel de «qui a de l'affinité pour l'eau», c'est-à-dire n'est pas susceptible de former une solution macroscopique diphasique dans de l'eau distillée à 25°C à une concentration supérieure à 1 % en poids.

Par unités B cationiques ou potentiellement cationiques, on entend des unités qui comprennent un groupe cationique ou potentiellement cationique. Les unités ou groupes cationiques sont des unités ou groupes qui présentent au moins une charge positive (généralement associée à un ou plusieurs anions comme l'ion chlorure, l'ion bromure, un groupe sulfate, un groupe méthylsulfate), quel que soit le pH du milieu dans lequel le copolymère est introduit. Les unités ou groupes potentiellement cationiques sont des unités ou groupes qui peuvent être neutres ou présenter au moins une charge positive selon le pH du milieu le copolymère est introduit. Dans ce cas on parlera d'unités potentiellement cationiques sous forme neutre ou sous forme cationique. Par extension on peut parler de monomères cationiques ou potentiellement cationiques.

Par unités $C_A$ anioniques ou potentiellement anioniques, on entend des unités qui comprennent un groupe anionique ou potentiellement anionique. Les unités ou groupes anioniques sont des unités ou groupes qui présentent au moins une charge négative (généralement associée à un ou plusieurs cations comme des cations de composés alcalins ou alcalino-terreux, par exemple le sodium, ou à un ou plusieurs composés cationiques comme l'ammonium), quel que soit le pH du milieu où est présent le copolymère. Les unités ou groupes potentiellement anioniques sont des unités ou groupes qui peuvent être neutres ou présenter au moins une charge négative selon le pH du milieu où est présent le copolymère. Dans ce cas on parlera de d'unités potentiellement anioniques $A_A$ sous forme neutre ou sous forme anionique. Par extension on peut parler de monomères anioniques ou potentiellement anioniques.

Par unités $C_N$ neutres, on entend des unités qui ne présentent pas de charge, quel que soit le pH du milieu où est présent le copolymère.

Dans la présente demande, sauf indications contraires, lorsqu'on parlera de masse molaire, il s'agira de la masse molaire moyenne en masse absolue, exprimée en g/mol. Celle-ci peut être déterminée par chromatographie de perméation de gel aqueux (GPC), par diffusion de lumière (DDL ou encore MALLS), avec un éluant aqueux ou un éluant organique (par exemple le diméthylacétamide, le diméthylformamide ...) selon la composition du polymère.

Dans la présente demande, on désigne par "charge moyenne Q d'un copolymère", la charge définie par l'équation suivante:

$$Q = \frac{[b]X_B - [c_A]X_{C_A}}{[b]X_B + [c_A]X_{C_A}}$$

où:

- [b] est la concentration molaire en unités B dans le copolymère,
- $[C_A]$ est la concentration molaire en unités $A_A$ dans la partie A,
- $X_B$ représente le taux de neutralisation éventuelle des unités B (dans le cas ou les unités B sont potentiellement cationiques); $X_B = [BH^+]/([B] + [BH^+])$,
- $X_{CA}$ représente le taux de neutralisation éventuelle des unités $C_A$ (dans le cas ou les unités $C_A$ sont potentiellement anioniques); $X_{CA} = [C_A^-]/([C_AH] + [C_A^-])$.

Dans la présente demande on désigne par "coacervat" un complexe du copolymère et d'un tensioactif. La présence de phases de coacervats peut être déterminée par les techniques connues se référant à ce type d'objets physico-chimiques. Par exemple, on peut mettre en oeuvre des analyses au microscope de la composition, ou de la composition diluée. Une phase de coacervat est identifiable comme une phase émulsifiée dans la composition, le cas échéant comme une phase supplémentaire après dilution ou modification de la composition (par exemple modification du pH). On peut utiliser des colorants pour distinguer les phases de coacervats d'autres phases dispersées dans la composition. Une méthode par mesure de transmittance est notamment décrite dans les exemples.

## Copolymère

Le copolymère selon l'invention comprend au moins deux types d'unités, A et B. Le polymère est de préférence un copolymère statistique, de préférence linéaire. Il peut également s'agir:

- d'un copolymère à gradient,
- d'un copolymère peigne,
- d'un copolymère à blocs comprenant un bloc comprenant les unités A et un bloc comprenant les unités B,
- d'un copolymère à blocs comprenant un bloc comprenant des unités A et B, et un bloc différent ne comprenant pas à la fois des unités A et des unités B, de préférence un bloc différent comprenant:
  - des unités $C_N$ non ioniques, hydrophiles ou hydrophobes, et/ou
  - des unités $C_A$ anioniques ou potentiellement anioniques.

Dans le cadre d'un copolymère peigne, le copolymère peut présenter un squelette comprenant les unités A et des chaînes macromoléculaires latérales présentant des unités B.

Le copolymère peut notamment comprendre en plus des unités A et B:

- des unités $C_N$ non ioniques, hydrophiles ou hydrophobes, et/ou
- des unités $C_A$ anioniques ou potentiellement anioniques.

Le rapport molaire entre les unités A et les unités B est compris entre 99/1 et 1/99, de préférence entre 95/5 et 5/95, plus préférablement entre 90/10 et 10/90. Selon un mode de réalisation, lé rapport entre les unités A et les unités B est supérieur à 50/50. Selon un autre mode, ce même rapport est inférieur à 50/50.

Les unités A et B représentent avantageusement de 1% à 100%, de préférence de 1 à 95% en moles des unités du copolymère. Selon un mode avantageux le copolymère comprend au moins 5% en moles d'unités $C_N$ non ioniques, hydrophiles ou hydrophobes, de préférence hydrophiles.

En général les charges (charges portées notamment par les unités B et A) présentes dans le copolymère sont portées par des groupes pendants.

<u>Unités A</u>

A titre de 1$^{er}$ groupe d'unités, le copolymère comprend des unités A comprenant un groupe sulfobétaïne qui comprend un groupe cationique et un groupe anionique soufré. Au sein de ces unités, le nombre de charges positives est égal au nombre de charges négatives. Les unités A sont électriquement neutres, dans au moins une gamme de pH. Cette charge anionique permanente peut être apportée par un ou des anions sulfonate. La charge cationique est apportée par un cation onium de la famille de l'azote (cation ammonium) ou du soufre (sulfonium, ...).

D'une manière préférentielle, les groupes bétaïnes sont les groupes bétaïnes des unités de A sont groupes pendants du copolymère (ils sont disposés en peigne le long de la chaîne macromoléculaire du polymère).

Les unités A, B, avec éventuellement d'autres unités, forment de préférence une chaîne hydrocarbonée polyalkylène (appelée aussi squelette) éventuellement interrompue par un ou plusieurs atomes d'azote ou de soufre.

Les groupes bétaïnes peuvent être reliés aux atomes de carbone d'une chaîne hydrocarbonée du polymère par l'intermédiaire notamment d'un motif hydrocarboné divalent ou polyvalent (par exemple alkylène ou arylène) éventuellement interrompu par un ou plusieurs hétéroatomes, d'oxygène notamment, un motif ester, un motif amide, ou bien par un lien valentiel.

Dans le copolymère, l'ensemble des unités comprenant un groupe bétaïne peut être constitué d'unités semblables ou différentes.

Le copolymère peut notamment être obtenu par polymérisation radicalaire en solution aqueuse de monomères comprenant des monomères pouvant mener aux unités B, et des monomères comprenant un groupe bétaïne éthyléniquement insaturé, notamment de monomères éthyléniquement insaturés portant au moins un groupe bétaïne de formule (I) à (IX) ci-dessus.

Lesdits monomères peuvent présenter, à titre d'exemple:

- un ou plusieurs radicaux hydrocarbonés mono- ou poly-éthyléniquement insaturés (notamment vinyle, allyle, styrényle ...),
- un ou plusieurs radicaux esters mono- ou poly-éthyléniquement insaturés (notamment acrylate, méthacrylate, maléate ...), et/ou
- un ou plusieurs radicaux amides mono- ou poly-éthyléniquement insaturés (notamment acrylamido, méthacrylamido...).

Les unités A présentes dans le copolymère selon l'invention derivent d'au moins un monomère bétaïne sélectionné dans le groupe constitué des monomères suivants:

- le sulfopropyl diméthyl ammonium éthyl méthacrylate, commercialisé par RASCHIG sous le nom SPE :

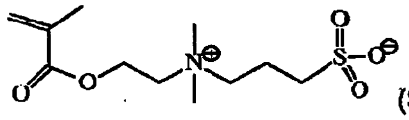

(SPE)

- le sulfoydroxypropyl diméthyl ammonium éthyl méthacrylate:

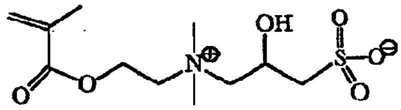

(SHPE)

- le sulfopropyl diméthylammonium propyl méthacrylamide, commercialisé par RASCHIG sous le nom SPP:

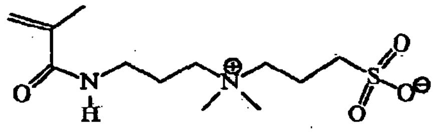

(SPP),

- le sulfohydroxypropyl diméthyl ammonium propyl méthacrylamido

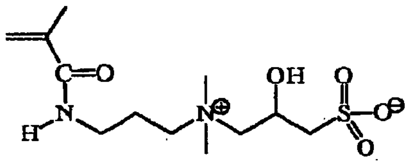

(SHPP)

Le polymère selon l'invention peut également être obtenu de manière connue par modification chimique d'un polymère dit polymère précurseur. Ainsi des unités sulfobétaïne peuvent être obtenues par modification chimique d'unités d'un polymère précurseur, de préférence par modification chimique d'un polymère comprenant des fonctions amines pendantes, à l'aide d'un composé électrophile sulfonaté, de préférence une sultone (propanesultone, butanesultone), un halogenoalkylsulfonate.

Quelques exemples de synthèse sont donnés ci-après:

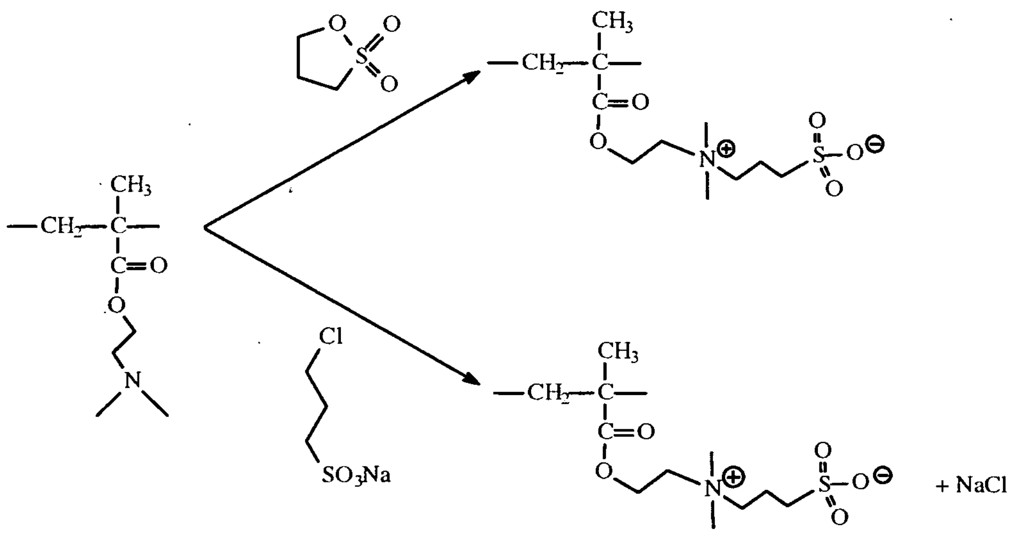

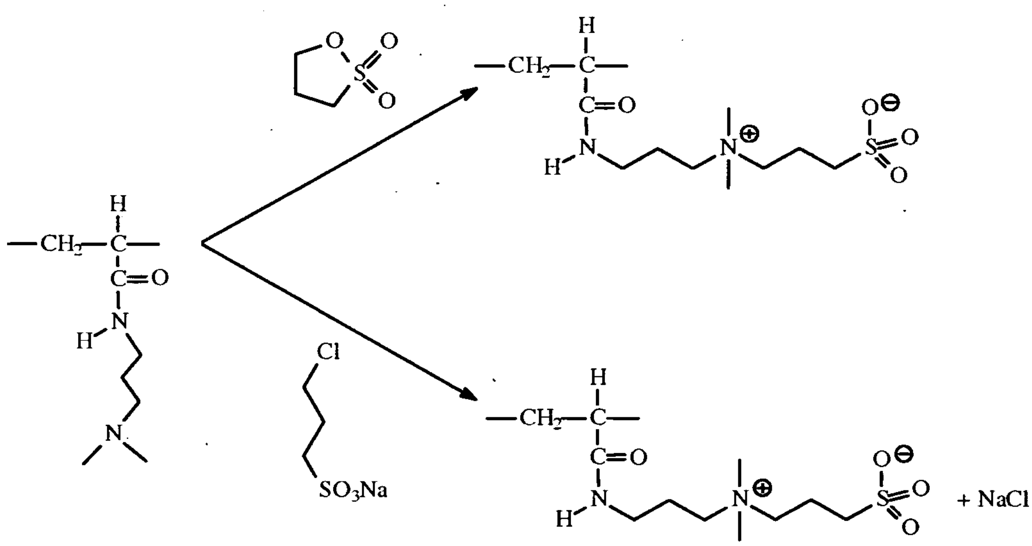

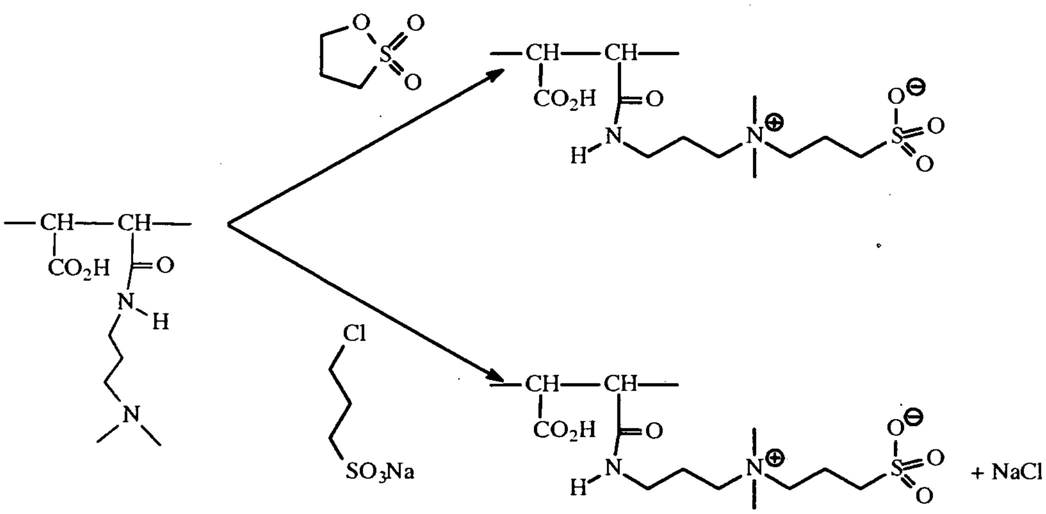

Les principales voies d'accès par modification chimique de polymère précurseur par les sultones et les halogenoalkylsufonates sont décrites notamment dans les documents suivants:

- "Synthesis and aqueous solution behaviour of copolymers containing sulfobetaine moieties in side chains", I.V. Berlinova, I.V. Dimitrov, R.G. Kalinova, N.G. Vladimirov, Polymer 41, 831-837 (2000)
- "Poly(sulfobetaine)s and corresponding cationic polymers: 3. Synthesis and dilute aqueous solution properties of poly(sulfobetaine)s derived from styrene-maleic anhydride)", Wen-Fu Lee and Chun-Hsiung Lee, Polymer 38 (4), 971-979 (1997)
- "Poly(sulfobetaine)s and corresponding cationic polymers. VIII. Synthesis and aqueous solution properties of a cationic poly(methyl iodide quaternized styrene-N,N-dimethylaminopropyl maleamidic acide) copolymer", Lee, Wen-Fu; Chen, Yan-Ming, Journal of Applied Polymer Science 80, 1619-1626 (2001)
- "Synthesis of polybetaines with narrow molecular mass distribution and controlled architecture", Andrew B. Lowe, Norman C. Billingham and Steven P. Armes, Chem. Commun., 1555-1556 (1996)
- "Synthesis and Properties of Low- Polydispersity Poly(sulfopropylbetaine)s and Their Block Copolymers", Andrew B. Lowe, Norman C. Billingham, and Steven P. Armes, Macromolecules 32,2141-2146 (1999)
- demande de brevet japonais publiée le 21 décembre 1999, sous le numéro 11-349826.

La préparation de polyphosphonato- et phosphinatobétaïnes par modification chimique est reportée dans «New polymeric phosphonato-, phosphinato- and carboxybétaïnes», T. Hamaide, Makromolecular Chemistry 187, 1097-1107 (1986).

Les unités A présentent l'une des formules suivantes:

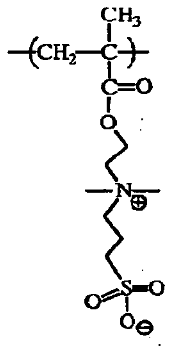

-(SPE)-

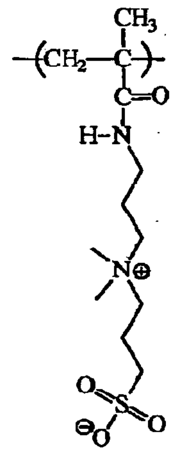

-(SPP)-

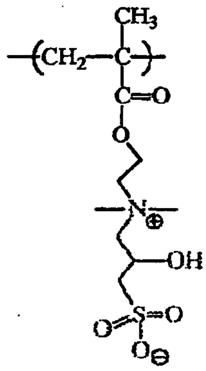

-(SHPE)-

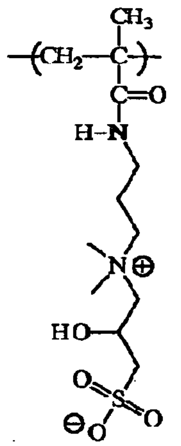

-(SHPP)-

Unités B

Les unités B sont des unités cationiques ou potentiellement cationiques comprenant au 1, 2, 3, ou plus groupes cationiques ou potentiellement cationiques, dans la chaîne formant le squelette du copolymère ou en position latérale par rapport à la chaîne formant le squelette du copolymère.

Les unités B cationiques sont des unités comprenant au moins un groupe ammonium quaternaire. Les unités B potentiellement cationiques peuvent être des unités comprenant au moins un groupe amine ternaire. A titre d'exemples de monomères potentiellement cationiques B dont peuvent dériver les unités B on peut mentionner:

- les N,N(dialkylaminoωalkyl)amides d'acides carboxyliques α-β monoéthyléniquement insaturés comme le N,N-diméthylaminométhyl -acrylamide ou -méthacrylamide, le 2(N,N-diméthylamino)éthyl-acrylamide ou - méthacrylamide, le 3(N,N-diméthylamino)propyl-acrylamide ou -méthacrylamide, le 4(N,N-diméthylamino)butyl-acrylamide ou -méthacrylamide
- les aminoesters α-β monoéthyléniquement Insaturés comme le 2(diméthyl amino)éthyl acrylate (ADAM), 2(diméthyl amino)éthyl méthacrylate (DMAM), le 3(diméthyl amino)propyl méthacrylate, le 2(tertiobutylamino)éthyl méthacrylate, le 2(dipentylamino)éthyl méthacrylate, le 2(diéthylamino)éthyl méthacrylate

- les vinylpyridines
- la vinyl amine
- les vinylimidazolines
- des monomères précurseurs de fonctions amines tels que le N-vinyl formamide, le N-vinyl acétamide, ... qui engendrent des fonctions amines primaires par simple hydrolyse acide ou basique.

A titre d'exemples de monomères cationiques B dont peuvent dériver les unités B, on peut mentionner:

- les monomères ammoniumacryloyles ou acryloyloxy comme

  - le chlorure de triméthylammoniumpropylméthacrylate,
  - le chlorure ou le bromure de triméthylammoniuméthylacrylamide ou méthacrylamide,
  - le méthylsulfate de triméthylammoniumbutylacrylamide ou méthacrylamide,
  - le méthylsulfate de triméthylammonlumpropylméthacrylamide (MES),
  - le chlorure de (3-méthaaylamidvpropyl)trirnéthylammonium (MAPTAC),
  - le chlorure de (3-acrylamidopropyl)triméthylammonium (APTAC),
  - le chlorure ou le méthylsulfate de méthacryloyloxyéthyl triméthylammonium,
  - le chlorure d'acryloyloxyéthyl triméthylammonium ; ou le méthylsulfate d'acryloyloxyéthyl triméthylammonium (ADAMQUAT Cl ou ADAMQUAT MeS),

- - le méthyle sulfate de méthyldiethylammonium éthyle acrylate (ADAEQUAT MeS),

  - le chlorure ou méthyle sulfate de benzyldimethylammonium éthyle acrylate (ADAMQUAT BZ 80),
  - le bromure, chlorure ou méthylsulfate de 1-éthyl 2-vinylpyridinium, de 1-éthyl 4-vinylpyridinium ;
  - les monomères N,N-dialkyldiallylamines comme le chlorure de N,N-diméthyldiallylammonium (DADMAC) ;
  - le chlorure de diméthylaminopropylméthacrylamide,N-(3-chloro-2-hydroxypropyl) triméthylammonium (DIQUAT chlorure),
  - le methylsulfate de diméthylaminopropylméthacrylamide,N-(3-methylsulfate-2-hydroxypropyl) triméthylammonium (DIQUAT methylsulfate)
  - le monomère de formule

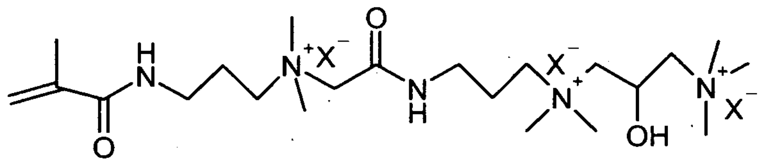

où $X^-$ est un anion, de préférence chlorure ou méthylsulfate.

A titre d'exemples de monomères potentiellement cationiques B dont peuvent dériver les unités B:

- les N,N(dialkylaminowalkyl)amides d'acides carboxyliques α-β monoéthyléniquement insaturés comme le N,N-diméthylaminométhyl -acrylamide ou -méthacrylamide, le 2(N,N-diméthylamino)éthyl-acrylamide ou - méthacrylamide, le 3(N,N-diméthylamino)propyl-acrylamide ou -méthacrylamide, le 4(N,N-diméthylamino)butyl-acrylamide ou -méthacrylamide
- les aminoesters α-β monoéthyléniquement insaturés comme le 2(diméthyl amino)éthyl acrylate (ADAM), 2(diméthyl amino)éthyl méthacrylate (DMAM), le 3(diméthyl amino)propyl méthacrylate, le 2(tertiobutylamino)éthyl méthacrylate, le 2(dipentylamino)éthyl méthacrylate, le 2(diéthylamino)éthyl méthacrylate
- les vinylpyridines
- la vinyl amine
- les vinylimidazolines
- des monomères précurseurs de fonctions amines tels que le N-vinyl formamide, le N-vinyl acétamide, ... qui engendrent des fonctions amines primaires par simple hydrolyse acide ou basique.

Unités C

A titre d'exemples de monomères non ioniques $C_N$ hydrophobes dont peuvent dériver les unités $C_N$ hydrophobes:

- les monomères vinylaromatiques tels que styrène, alpha-méthylstyrène, vinyltoluène...
- les halogénures de vinyle ou de vinylidène, comme le chlorure de vinyle, chlorure de vinylidène
- les $C_1$-$C_{12}$ alkylesters d'acides α-β monoéthyléniquement insaturés tels que les acrylates et méthacrylates de méthyle, éthyle, butyle, acrylate de 2-éthylhexyle ...
- les esters de vinyle ou d'allyle d'acides carboxyliques saturés tels que les acétates, propionates, versatates, stéarates ... de vinyle ou d'allyle
- les nitriles α-β monoéthyléniquement insaturés contenant de 3 à 12 atomes de carbone, comme l'acrylonitrile, le methacrylonitrile ...
- les α-oléfines comme l'éthylène ...
- les diènes conjugués, comme le butadiène, l'isoprène, le chloroprène,
- le diethyleneglycolethyletheracrylate ou le diethyleneglycolethylethermethacrylate.

A titre d'exemples de <u>monomères non ioniques hydrophiles $C_N$</u> dont peuvent dériver les unités $C_N$ non ioniques hydrophiles, on peut mentionner:

- les hydroxyalkylesters d'acides α-β éthyléniquement insaturés comme les acrylates et méthacrylates d'hydroxyéthyle, d'hydroxypropyle, le glycérol monométhacrylate...
- les amides α-β éthyléniquement insaturés comme l'acrylamide (AM), le méthacrylamide, le N-méthylolacrylamide, le diméthylacylamide, le diméthylméthacrylamide, ...
- les monomères α-β éthyléniquement insaturés portant un segment polyoxyalkyléné hydrosoluble du type polyoxyde d'éthylène, comme les polyoxyde le cas échéant statistique ou à blocs d'éthylène et/ou de propylène α-méthacrylates (BISOMER S20W, S10W, ... de LAPORTE) ou α,ω-diméthacrylates, le SIPOMER BEM de RHODIA (méthacrylate de polyoxyéthylène ω-béhényle éventuellement en mélange), le SIPOMER SEM-25 de RHODIA (méthacrylate de polyoxyéthylène ω-tristyrylphényle) ...
- les monomères α-β éthyléniquement insaturés précurseurs d'unités ou de segments hydrophiles tels que l'acétate de vinyle qui, une fois polymérisés, peuvent être hydrolysés pour engendrer des unités alcool vinylique ou des segments alcool polyvinylique
- les vinylpyrrolidones
- les monomères α-β éthyléniquement insaturés de type uréido et en particulier le méthacrylamido de 2-imidazolidinone éthyle éventuellement en mélange (Sipomer WAM II de RHODIA)
- le nonethyleneglycolmethyletheracrylate ou le nonethyleneglycolmethylethermethacrylate.

A titre d'exemples de <u>monomères anioniques ou potentiellement anioniques $C_A$,</u> dont peuvent dériver des unités $C_A$ anioniques ou potentiellement anioniques, on peut mentionner :

- des monomères possédant au moins une fonction carboxylique, comme les acides carboxyliques α-β éthyléniquement insaturés ou les anhydrides correspondants, tels que les acides ou anhydrides acrylique, méthacrylique, maleique, l'acide fumarique, l'acide itaconique, le N-méthacroyl alanine, le N-acryloylglycine et leurs sels hydrosolubles
- des monomères précurseurs de fonctions carboxylates, comme l'acrylate de tertiobutyle, qui engendrent, après polymérisation, des fonctions carboxyliques par hydrolyse.
- des monomères possédant au moins une fonction sulfate ou sulfonate, comme le 2-sulfooxyethyl méthacrylate, l'acide vinylbenzène sulfonique, l'acide allyl sulfonique, le 2-acrylamido-2méthylpropane sulfonique, l'acrylate ou le méthacrylate de sulfoethyle , l'acrylate ou le méthacrylate de sulfopropyle et leurs sels hydrosolubles
- des monomères possédant au moins une fonction phosphonate ou phosphate, comme l'acide vinylphosphonique,... les esters de phosphates éthyléniquement insaturés tels que les phosphates dérivés du méthacrylate d'hydroxyéthyle (Empicryl 6835 de RHODIA) et ceux dérivés des méthacrylates de polyoxyalkylènes et leurs sels hydrosolubles.

Selon des modes de réalisation intéressants, le copolymère est:

- d'un copolymère dérivant de

    - A: SPE, de préférence de 5 à 95 mol%, plus préférablement de 10 à 90 mol%, et
    - B: MAPTAC, de préférence de 5 à 95 mol%, plus préférablement de 10 à 90 mol%,

- d'un copolymère dérivant de

  - A: SPE, de préférence de 5 à 95 mol%, plus préférablement de 10 à 90 mol%, et
  - B: DIQUAT, de préférence de 5 à 95 mol%, plus préférablement de 10 à 90 mol%,

- d'un copolymère dérivant de

  - A: SPE, de préférence de 5 à 90 mol%, plus préférablement de 10 à 80 mol%,
  - B: MAPTAC, de préférence de 5 à 90 mol%, plus préférablement de 10 à 80 mol%, et
  - C: acrylamide, de préférence de 5 à 90 mol%, plus préférablement de 10 à 80 mol%,

- d'un copolymère dérivant de

  - A: SPE, de préférence de 5 à 90 mol%, plus préférablement de 10 à 80 mol%,
  - B: DIQUAT, de préférence de 5 à 90 mol%, plus préférablement de 10 à 80 mol%, et
  - C: acrylamide, de préférence de 5 à 90 mol%, plus préférablement de 10 à 80 mol%,

- d'un copolymère dérivant de

  - A: SPP, de préférence de 5 à 95 mol%, plus préférablement de 10 à 90 mol%, et
  - B: MAPTAC, de préférence 5 à 95 mol%, plus préférablement de 10 à 90 mol%,

- d'un copolymère dérivant de

  - A: SPP, de préférence de 5 à 95 mol%, plus préférablement de 10 à 90 mol%, et
  - B: DIQUAT, de préférence de 5 à 95 mol%, plus préférablement de 10 à 90 mol%,

- d'un copolymère dérivant de

  - A: SPP, de préférence de 5 à 90 mol%, plus préférablement de 10 à 80 mol%,
  - B: MAPTAC, de préférence de 5 à 90 mol%, plus préférablement de 10 à 80 mol%, et
  - C: acrylamide, de préférence de 5 à 90 mol%, plus préférablement de 10 à 80 mol%, ou

- d'un copolymère dérivant de

  - A: SPP, de préférence de 5 à 90 mol%, plus préférablement de 10 à 80 mol%,
  - B: DIQUAT, de préférence de 5 à 90 mol%, plus préférablement de 10 à 80 mol%, et
  - C: acrylamide, de préférence de 5 à 90 mol%, plus préférablement de 10 à 80 mol%.

La masse molaire moyenne en masse du copolymère est de préférence comprise entre 5000 g/mol et 400000 g/mol (valeur relative, calibrée en GPC aqueuse avec des étalons de polyoxyde d'éthylène). La masse molaire moyenne en masse absolue peut être de préférence comprise entre 10000 et 4000000 g/mol.

La masse molaire moyenne en masse absolue est avantageusement supérieure ou égale à 100000 g/mol, de préférence supérieure ou égale à 250000 g/mol. La masse molaire moyenne en masse peut notamment être'inférieure ou égale à 2000000 g/mol, ou même à 1000000 g/mol. Des polymères de relativement haute masse peuvent améliorer le traitement de la surface par exemple par un dépôt supérieur. Dans le cadre de polymérisations radicalaires, mettant en présence les monomères et un initiateur de radicaux libre (ou "amorceur"), souvent en solution aqueuse et à température appropriée, la masse molaire moyenne peut être contrôlée par la quantité d'initiateur utilisée: moins il est utilisé d'initiateur, plus la masse molaire moyenne est élevée. On pourra notamment utiliser moins de 1 % en moles, voir même moins de 0,5% en moles, par exemple de 0,01 à 0.25% en moles, par rapport aux monomères engagés d'initiateur.

De préférence le copolymère est hydrosoluble ou hydrodispersable.

Le copolymère selon l'invention peut être présenté notamment sous forme de poudre, sous forme de dispersion dans un liquide ou sous forme de solution dans un solvant (eau ou autre). La forme dépend généralement des exigences liées à l'utilisation du copolymère. Elle peut être aussi liée au procédé de préparation du copolymère.

<u>Compositions</u>

Le copolymère peut être utilisé dans une composition, typiquement une composition destinée à être appliquée sur une surface.

Le copolymère peut ainsi être utilisé dans les domaines des compostions cosmétiques, des compositions pour

les soins ménagers (détergence, lessives, liquide vaisselles, produits pour les lave-vaisselle, produits de nettoyages des surfaces dures, etc) des compositions pour les soins (notamment nettoyage) industriels ou institutionnels, des compositions de traitement de surfaces visant à en modifier les propriétés telles que l'hydrophilie, l'adhésion, des compositions mises en oeuvre dans les techniques d'extraction de pétrole ou de gaz, des compositions de peinture, des compositions phytosanitaires, des revêtements ou traitements industriels ou professionnels destinés à faciliter un nettoyage un ultérieur, des compositions de traitements des métaux ou des plastiques.

Le copolymère peut en outre être utilisé en tant qu'agent de floculation, ou agent de dépôt sur une surface ou agent d'aide au dépôt sur une surface.

Ainsi des compositions utiles peuvent être des compositions pour le traitement ou la modification de surfaces comprenant:

- un vecteur de préférence liquide, par exemple un vecteur cosmétiquement acceptable,
- le copolymère,
- éventuellement un tensioactif,
- éventuellement un sel, un acide et/ou une base, et
- éventuellement un agent pour le traitement ou la modification de la surface.

La quantité en poids de copolymère (en poids de matière sèche) par rapport à la composition est de préférence supérieure à 0,001 %, de préférence supérieure à 0,01 %, par exemple de l'ordre de 0,1 à 0,9%. Elle peut être est inférieure à 10%, souvent inférieure à 5% et même à 1%. La quantité en poids peut dépendre de la nature du traitement de surface ou de la modification.

Avantageusement la composition comprend un tensioactif anionique ou amphotère. La composition peut comprendre un tensioactif non ionique. Elle peut comprendre un mélange ou une association de tensioactifs anioniques, amphotères et/ou non ioniques.

Avantageusement les unités B sont sous forme cationiques au pH de la composition.

Selon un mode de réalisation préférentiel la composition:

- comprend un tensioactif anionique ou amphotère, et
- comprend des coacervats ou elle forme des coacervats par dilution et/ou modification du pH.

Pour cela le copolymère, le tensioactif anionique ou amphotère, le vecteur, éventuellement d'autres tensioactifs, un sel, une base et/ou acide, et leurs quantités, sont tels que au moins une partie de système constitué du copolymère, du tensioactif anionique ou amphotère, du vecteur, et éventuellement les autres tensioactifs, sel, base et/ou acide

- forme des coacervats par dilution et/ou modification du pH, ou
- comprend des coacervats.

L'agent pour le traitement ou la modification de la surface peut par exemple être un polyorganosiloxane, un actif anti-pelliculaire, un parfum, une huile, ou un filtre UV.

En ce qui concerne la composition il peut notamment s'agir:

- d'une composition cosmétique, de préférence destinée à être rincée, de préférence un shampooing, un produit de mise en forme des cheveux, un après-shampooing, un produit de soin des cheveux, un produit de soin de la peau ou un gel douche,
- d'une composition détergente ou rinçante pour les soins ménagers ou industriels ou institutionnels, de préférence pour le soin du linge ou pour le nettoyage ou le traitement de surfaces dures.

La composition peut être utilisée dans un procédé de traitement ou de modification d'une surface, comprenant les étapes suivantes:

- appliquer sur la surface la composition, et
- éventuellement, éliminer le vecteur ou diluer la composition ou modifier le pH.

Quand la surface est la peau ou les cheveux, le traitement ou la modification peuvent par exemple être un conditionnement de la peau ou du cheveux.

Dans le domaine des soins ménagers industriels ou institutionnels, la surface peut notamment être une surface textile (la composition pouvant être une lessive ou un adoucissant par exemple) ou une surface dure.

La surface peut également être une surface de type non tissée, par exemple une surface présente dans des

produit d'hygiène domestique tels que des lingettes, et/ou une surface présente dans des produits d'hygiène personnelle, telle qu'une surface textile entrant des la confection de couches pour bébés, de produits de protection féminine ou de produits pour l'incontinence adulte.

Composition cosmétique

Quand le copolymère est utilisé dans une composition (ou "formulation") cosmétique, il peut aider ou contribuer à un dépôt de matière (effet conditionneur), et/ou, plus généralement, à optimiser des effets cosmétiques tels la douceur, la souplesse, le démêlage, la brillance, l'aptitude au coiffage sur cheveux secs ou mouillés. Il peut aider à la conception de formulations faciles à préparer, faciles mettre en oeuvre, et suffisamment stables.

En outre, il peut contribuer à proposer de nouvelles compositions cosmétiques, en particulier destinées à être rincées, présentant des qualités améliorées en matière de stabilité et/ou de simplification des formulations et/ou de transparence et/ou de qualités cosmétiques (mentionnées ci-dessus) et/ou de dépôt de matière (dépôt du copolymère ou dépôt d'autres matières comme des huiles minérale, végétales ou synthétiques, par exemples de huiles silicones, ou "polyorganosiloxanes").

Les compositions cosmétiques sont de préférence des compositions destinées à être rincées. Il peut s'agir par exemple d'un shampooing, d'un gel-douche ou d'un après-shampooing. Il peut néanmoins s'agir d'une composition de soin capillaire qui n'est pas destinée à être rincée, par exemple un après shampooing destiné à ne pas être rincé, un lait démêlant, une eau démêlante, une eau de lissage, un revêtement de cuticule, un produit de soin capillaire coiffant et/ou recoiffant, un produit de protection solaire, une crème de soin, un démaquillant, un maquillage, des lingettes démaquillantes ou hydratantes, des mousses à raser, des mousses coiffantes ou fixantes.

L'invention concerne aussi l'utilisation du copolymère dans les compositions cosmétiques.

Les compositions, lorsqu'elles comprennent un agent conditionneur, par exemple une silicone (appelée de manière équivalente un polyorganosiloxane), favorisent le dépôt dudit agent. Le copolymère aide ainsi au dépôt d'agents conditionneurs, plus particulièrement de silicones (ou polyorganosiloxanes). En outre, les compositions comprenant un polyorganosiloxane et le copolymère présentent d'excellentes propriétés conditionnantes, pour les cheveux ou la peau, ainsi que des propriétés sensorielles ou cosmétiques intéressantes, qui peuvent être recherchées par les consommateurs. Ainsi elles peuvent procurer un profil intéressant de douceur, de souplesse, de volume, de démêlage, d'aptitude au coiffage sur cheveux mouillé et/ou d'aptitude au coiffage sur cheveux sec. Ces effets peuvent rendre plus simples les formulations et/ou moins coûteuses. Les compositions présentent en outre des propriétés de moussage satisfaisantes, notamment en eau dure.

Les compositions cosmétiques peuvent comprendre avantageusement de 0,01 % à 5% en poids du copolymère, de préférence de 0,05 à 1,5 % en poids, par exemple de 0,1 à 0,5% en poids. On précise que le copolymère peut être introduit dans la composition sous forme d'une solution aqueuse plus ou moins concentrée. Les quantités mentionnées ci-dessus sont des quantités exprimées en extrait sec.

Vecteur cosmétiquement acceptable des compositions cosmétiques

Tout vecteur cosmétiquement acceptable permettant de formuler le copolymère ampholyte et d'obtenir la forme de composition cosmétique désirée, pour l'utilisation visée, peut être utilisé. Différents vecteurs cosmétiquement acceptables pour différents types de formulations sont connus de l'homme du métier.

A titre d'exemples de vecteurs cosmétiquement acceptables, on peut citer les vecteurs aqueux (comprenant de l'eau), les vecteurs alcooliques (comprenant un alcool, par exemple de l'éthanol, l'isopropanol, l'éthylène glycol ou les polyéthylène glycol), le propylène glycol, les vecteurs hydro-alcooliques (comprenant un mélange d'eau et d'un alcool par exemple de l'éthanol, l'isopropanol, l'éthylène glycol ou les polyéthylène glycol). Certaines huiles, volatiles ou non, peuvent également être utilisées. On cite par exemple les silicones fluides, tels que le cyclopentasiloxane, par exemple le Mirasil CM5 commercialisé par Rhodia.

L'homme du métier sait choisir les vecteurs adaptés aux types de formulations souhaités, et aux utilisations visées. Par exemple des vecteurs aqueux sont généralement utilisés pour des shampooings ou gels-douche. Un vecteur propylène glycol peut être utilisé pour des compositions sous forme de crèmes. Un vecteur cyclométhicone peut être utilisé pour des compositions de maquillage, par exemple pour des fonds de teint.

Tensioactifs dans les compositions cosmétiques

La composition est une composition aqueuse comprenant éventuellement des tensioactifs. Il peut s'agir d'un mélange de différents tensioactifs. Les tensioactifs compris dans la composition comprennent de préférence au moins un tensioactif anionique. Les tensioactifs peuvent également comprendre des tensioactifs amphotères (amphotères vrais ou zwitterioniques), des tensioactifs neutres (tensioactifs non ioniques) et/ou des tensioactifs cationiques. Les

compositions comprenant au moins un tensioactif anionique et au moins un tensioactif amphotère sont particulièrement avantageuses, notamment pour des raisons de douceur. La teneur totale en tensioactifs dans la composition est généralement comprise entre 0 et 30% en poids.

Pour des compositions destinées au traitement des cheveux, comme des shampooings, la teneur en tensioactif est avantageusement comprise entre 10 et 20% en poids. De telles compositions peuvent comprendre des sels, par exemple du chlorure de sodium ou d'ammonium, avantageusement en teneur inférieure à 3% en poids.

Pour des compositions destinées au traitement de la peau, comme des gels-douche, la teneur en tensioactif est avantageusement comprise entre 5 et 15% en poids. De telles compositions comprennent également de préférence au moins 2% en poids de sels, par exemple du chlorure de sodium ou d'ammonium.

Pour des après-shampooings, la teneur en tensioactifs peut être inférieure à 5% en poids.

La proportion en poids de tensioactifs anioniques par rapport à l'ensemble des tensioactifs est de préférence supérieure à 50%, de préférence supérieure à 70%.

<u>Paramètres (pH) utiles pour les compositions cosmétiques</u>

Le pH de la composition peut être est supérieur ou égal à 5,5. Il est par exemple compris entre 5,5 et 7,5, de préférence entre 6 et 6,5. Toutefois le pH de la composition peut être de 3,5 à moins de 5,5, de préférence compris entre 4,5 et moins de 5,5, de préférence de 5 à moins de 5,5. Le pH dépend évidemment des composés présents dans la composition. On peut évidemment utiliser dans la composition des agents de régulation du pH, acides ou bases, par exemple de l'acide citrique, ou de l'hydroxyde de sodium, de potassium ou d'ammonium.

<u>Natures des tensioactifs pour les compositions cosmétiques</u>

Les <u>tensioactifs anioniques</u> peuvent être choisis parmi les tensioactifs suivants:

- les alkylesters sulfonates, par exemple de formule $R-CH(SO_3M)-CH_2COOR'$, ou les alkylesters sulfates, par exemple de formule $R-CH(OSO_3M)-CH_2COOR'$, où R représente un radical alkyle en $C_8$-$C_{20}$, de préférence en $C_{10}$-$C_{16}$, R' un radical alkyle en $C_1$-$C_6$, de préférence en $C_1$-$C_3$ et M un cation alcalino-terreux, par exemple sodium, ou le cation ammonium. On peut citer tout particulièrement les méthyl ester sulfonates dont le radical R est en $C_{14}$-$C_{16}$;
- les alkylbenzènesulfonates, plus particulièrement en $C_9$-$C_{20}$, les alkylsulfonates primaires ou secondaires, notamment en $C_8$-$C_{22}$, les alkylglycérol sulfonates ;
- les alkylsulfates par exemple de formule $ROSO_3M$, où R représente un radical alkyle ou hydroxyalkyle en $C_{10}$-$C_{24}$, de préférence en $C_{12}$-$C_{20}$ ; M un cation de même définition que ci-dessus ;
- les alkyléthersulfates par exemple de formule $RO(OA)_nSO_3M$ où R représente un radical alkyle ou hydroxyalkyle en $C_{10}$-$C_{24}$, de préférence en $C_{12}$-$C_{20}$ ; OA représentant un groupement éthoxylé et/ou propoxylé ; M représentant un cation de même définition que ci-dessus, n variant généralement de 1 à 4, comme par exemple le lauryléthersulfate avec n = 2 ;
- les alkylamides sulfates, par exemple de formule $RCONHR'OSO_3M$ où R représente un radical alkyle en $C_2$-$C_{22}$, de préférence en $C_6$-$C_{20}$, R' un radical alkyle en $C_2$-$C_3$, M représentant un cation de même définition que ci-dessus, ainsi que leurs dérivés polyalcoxylés (éthoxylés et/ou propoxylés) (alkylamidoether sulfates
- les sels d'acides gras saturés ou insaturés, par exemple comme ceux en $C_8$-$C_{24}$, de préférence en $C_{14}$-$C_{20}$ et d'un cation alcalino-terreux, les N-acyl N-alkyltaurates, les alkyliséthionates, les alkylsuccinamates et alkylsulfo succinates, les monoesters ou diesters de sulfosuccinates, les N-acyl sarcosinates, les polyéthoxycarboxylates ;
- les mono et di esters phosphates, par exemple de formule suivante : $(RO)_x$-$P(=O)(OM)_x$ ou R représente un radical alkyle, alkylaryle, arylalkyle, aryle, éventuellement polyalcoxylés, x et x' étant égaux à 1 ou 2, à la condition que la somme de x et x' soit égale à 3, M représentant un cation alcalino-terreux ;

Les <u>tensioactifs non ioniques</u> peuvent être choisis parmi les tensioactifs suivants:

- les alcools gras alcoxylés ;
- les triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés
- les alkyls phénols alcoxylés
- les produits résultant de la condensation de l'oxyde d'éthylène avec un composé hydrophobe résultant de la condensation de l'oxyde de propylène avec le propylène glycol, tels les Pluronic commercialisés par BASF ;
- les produits résultant de la condensation de l'oxyde d'éthylène le composé résultant de la condensation de l'oxyde de propylène avec l'éthylènediamine, tels les Tetronic commercialisés par BASF ;
- les alkylpolyglycosides comme ceux décrits dans US 4565647 ;
- les amides d'acides gras par exemple en $C_8$-$C_{20}$ ;

Les <u>tensioactifs amphotères</u> (amphotères vrais comprenant un groupe ioniques et un groupe potentiellement ionique de charge opposée, ou zwitterioniques comprenant simultanément deux charges opposées) peuvent être choisis parmi les tensioactifs suivants:

- les bétaïnes de manière générale, notamment carboxybétaïnes de par exemple la lauryl bétaïne (Mirataine BB de la société Rhodia) ou l'octylbétaïne; les amidoalkylbétaïnes, comme la cocamidopropyl bétaïne (CAPB) (Mirataine BDJ de la société Rhodia Chimie) ;
- les sulfo-bétaïnes ou sultaines comme la cocamidopropyl hydroxy sultaïne (Mirataine CBS de la société Rhodia) ;
- les alkylamphoacétates et alkylamphodiacétates, comme par exemple comprenant une chaîne coco, lauryle (Miranol C2M, C32, L32 notamment, de la société Rhodia) ;
- les alkylamphopropionates ou les alkylamphodipropionates, (Miranol C2M SF) ;
- les alkyl amphohydroxypropyl sultaïnes (Miranol CS).

Les <u>tensioactifs cationiques</u> peuvent être choisis parmi les sels d'amines grasses primaires, secondaires ou tertiaires, éventuellement polyethoxylées, les sels d'ammonium quaternaires tels que les chlorures ou les bromures de tetraalkylammonium, d'alkylamidoalkylammonium, de trialkylbenzylammonium, de trialkylhydroxyalkylammonium, ou d'alkylpyridinium, les dérivés d'imidazoline, les oxydes d'amines à caractère cationique.

A titre d'exemples de compositions utiles, on peut citer:

- Les compositions «sodium» pour shampooings comprenant typiquement 12 à 16% en poids d'alkylethersulfate de sodium (par exemple laurylethersulfate de sodium « SLES ») ou d'un mélange d'alkylethersulfate de sodium et d'alkylsulfate de sodium (par exemple laurylsulfate de sodium «SLS»), 1 à 3 % d'un tensioactif amphotère (par exemple cocoamidopropylbétaïne « CAPB »), 0.5 à 2 % d'un sel (par exemple chlorure de sodium).
- Les compositions «ammonium» pour shampooings comprenant typiquement 12 à 16% en poids d'alkylethersulfate d'ammonium (par exemple laurylethersulfate d'ammonium « ALES ») ou d'un mélange d'alkylethersulfate d'ammonium et d'alkylsulfate d'ammonium (par exemple laurylsulfate d'ammonium «ALS»), 1 à 3 % d'un tensioactif amphotère (par exemple cocoamidopropylbétaïne « CAPB »), 0 à 2 % d'un sel (par exemple chlorure d'ammonium).
- Les compositions «sodium» pour gel-douche comprenant typiquement 6 à 10% en poids d'alkylethersulfate de sodium (par exemple laurylethersulfate de sodium « SLES ») ou d'un mélange d'alkylethersulfate de sodium et d'alkylsulfate de sodium (par exemple laurylsulfate de sodium «SLS»), 1 à 3 % d'un tensioactif amphotère (par exemple cocoamidopropylbétaïne « CAPB »), 2 à 4 % d'un sel (par exemple chlorure de sodium).
- Les compositions «sodium» pour gel-douche comprenant typiquement 6 à 10% en poids d'alkylethersulfate d'ammonium (par exemple laurylethersulfate d'ammonium « ALES ») ou d'un mélange d'alkylethersulfate d'ammonium et d'alkylsulfate d'ammonium (par exemple laurylsulfate d'ammonium «ALS»), 1 à 3 % d'un tensioactif amphotère (par exemple cocoamidopropylbétaïne « CAPB »), 0 à 4 % d'un sel (par exemple chlorure d'ammonium).

<u>Autres composés pouvant être présents dans les compositions cosmétiques</u>

La composition peut comprendre tout autre composé utilisé dans les compositions cosmétiques destinées à être rincées (shampooing, gel-douche, après-shampooings...), ou destinées à ne pas être rincées.

On cite par exemple les séquestrants, les adoussissants, les modificateurs de mousse, les colorants, les agents nacrant (pearlizers), les agents hydratants, les agents antipelliculaires ou antiséborrhéiques, les agents de mise en suspension, les agents de mise en émulsion, les céramides, les pseudocéramides, les éléctrolytes, les acides gras, les esters d'acides gras; les hydroxyacides, les épaississants, les parfums, les conservateurs, les filtres solaires organiques ou minéraux, les protéines, les vitamines, des polymères, des silicones "polyorganosiloxanes", des agents de stabilisation et/ou de conditionnement et/ou d'aide au conditionnement, autres que le copolymère ampholyte et que les polyorganosiloxanes, notamment des polymères. Certains de ces composés sont détaillés ci-dessous.

<u>Agent de stabilisation et/ou de conditionnement et/ou d'aide au conditionnement</u>

La composition cosmétique selon l'invention peut avantageusement comprendre au moins un agent de stabilisation et/ou de conditionnement (agents conditionneurs) et/ou d'aide au conditionnement. On parle aussi parfois

d'agents de suspension. Par aide au conditionnement, on entend que la présence de l'agent améliore le conditionnement lié à d'autres' composés, par exemple des huiles ou silicones. Les agents sont entendus comme des agents différents du polyorganosiloxane de formule (I). De tels agents sont connus de l'homme du métier. La composition selon l'invention peut comprendre plusieurs de ces agents (mélanges ou combinaisons), pour associer leurs effets et/ou créer des synergies. Par ailleurs, certains agents peuvent exercer plusieurs fonctions. C'est le cas par exemple des polysaccharides, et leurs dérivés cationiques, par exemple des dérivés cationiques de guars.

La proportion en poids de tels agents peut être typiquement de 0,1% à 10% en poids, de préférence de 0,3% à 8% en poids, pour des polysaccharides ou d'autres agents.

A titre d'exemples d'agents de stabilisation (ou agents stabilisants), particulièrement utiles pour des compositions comprenant des polyorganosiloxanes, on peut citer:

- les polyacrylates réticulés, par exemple les polymères de type CARBOPOL ou CARBOMER commercialisés par BF Goodrich ou Noveon, ACRITAMER commercialisés par RITA ou TEGO CARBOMER commercialisés par Goldschmidt. Ces composés peuvent être typiquement présents en quantité de 0,1 à 3%, de préférence de 0,3 à 2%, en poids par rapport à la composition.
- les copolymères des acrylates/aminoacrylates/ itaconates PEG 20 alkyles $C_{10}$-$C_{30}$ commercialisés par National Starch sous le nom STRUCTURE PLUS. Ces composés peuvent être typiquement présents en quantité de 0,1 à 3%, de préférence de 0,3 à 2%, en poids par rapport à la composition.
- les solides insolubles formant un réseau dans la composition. Il peut s'agir de mono et/ou di-esters d'acides gras d'éthylène glycol, les acides gras étant de préférence en $C_{16}$-$C_{18}$. Il peut en particulier s'agir d'éthylène glycol distéarate (EGDS), par exemple commercialisé par Rhodia en concentré avec d'autres ingrédient sous le nom MIRASHEEN. Ce composé peut être typiquement présent en quantité de 3 à 10%, de préférence de 5 à 8% en poids par rapport à la composition.

On peut citer également des agents viscosants, gelifiants ou texturants comme les copolymères acryliques anioniques de type ACULYNE commercialisés par ISP ou Rohm & Haas), les polysaccharides et leurs dérivés non cationiques tels que les dérivés de la cellulose comme l'hydroxypropylcellulose, la carboxyméthylcellulose, les dérivés non-ioniques de guars comme l'hydroxypropyl guar (par exemple les Jaguar HP commercialisé par Rhodia), la caroube, la gomme de tara ou de cassia, la gomme xanthane (par exemple les Rhodicare commercialisés par Rhodia), les succinoglycanes (par exemple le Rheozan commercialisé par Rhodia), les alginates, les carraghénannes, les dérivés de la chitine ou tout autre polysaccharide à fonction texturante. Ces polysaccharides et leurs dérivés peuvent être incorporés seuls ou en combinaison synergique à d'autres polysaccharides. Ces composés peuvent être typiquement présents en quantité de 0,1 à 3%, de préférence de 0,3 à 1%, en poids par rapport à la composition.

A titre d'exemples d'agents de stabilisation et/ou d'agents de conditionnement et/ou d'aide ou conditionnement, on peut citer:

- les polymères cationiques dérivés de polysaccharides, par exemple les dérivés cationiques de celluloses, les dérivés cationiques d'amidons, les dérivés cationiques de guars, les dérivés cationique de caroube.
- les polymères cationiques synthétiques,
- les mélanges ou combinaisons de ces agents.

Les polymères cationiques, synthétiques ou non, pouvant assurer une fonction d'agent de conditionnement, sont notamment des polymères de type polyquaternium, comme par exemple les polyquaternium-1, polyquaternium-2, polyquaternium-4, polyquaternium-5, polyquaternium-6 (également connu comme Merquat 1000 disponible auprès de Nalco), polyquaternium-7 (également connu comme Merquat 5500 disponible auprès de Nalco), polyquaternium-8, polyquaternium-9, polyquaternium10 (également connu comme Polymer JR 400, commercialisé par Amercol), polyquaternium-11, polyquaternium-12, polyquaternium-13, polyquaternium-14, polyquaternium-15, polyquaternium-16, polyquaternium-17, polyquaternium-18, polyquaternium-19, polyquaternium-20, polyquaternium-22 (également connu comme Merquat 280, 281, 298 disponibles auprès de Nalco), polyquaternium-24, polyquaternium-27, polyquaternium-28, polyquaternium-29 (également connu comme Kytamer KCO disponible auprès de Amerchol), polyquaternium-30, polyquatemium-31, polyquatemium-32, polyquaternium-33, polyquaternium-34, polyquaternium-35, polyquatemium-36, polyquaternium-37, polyquaternium-39 (également connu comme Merquat 3300, 3331 disponibles auprès de Nalco), polyquaternium-44, polyquaternium-27 (également connu comme Merquat 2001 disponible auprès de Nalco) et polyquaternium-55.

Comme mentionné ci-dessus la composition peut comprendre d'autres polymères, synthétiques ou naturels ou issus de procédé de préparation biologiques, le cas échéant fonctionnalisés, par exemple par des groupe cationiques ou neutres. Ces polymères peuvent avoir une action de stabilisation, de structuration des compositions, et/ou une action de conditionneur (dépôt à la surface de la peau ou des cheveux).

On cite à titre d'exemples les dérivés cationiques de polysaccharides, comme les dérivés de guar ou de cellulose. Des polymères cationiques fonctionnalisés par des groupes hydrophobes comme des chaînes alkyles en C1-C14, de préférence en C2-C8, présentant éventuellement un groupement hydroxyle, peuvent être utilisés. Ces groupes hydrophobes sont rattachés à la chaîne polymérique principale par l'intermédiaire de liaisons éthers.

Par ailleurs, et dans le cas des guars cationiques modifiés hydrophobes ou non, le groupement cationique est un groupement ammonium quaternaire portant trois radicaux, identiques ou non, choisis parmi l'hydrogène, un radical alkyle comprenant 1 à 22 atomes de carbone, plus particulièrement 1 à 14, de manière avantageuse 1 à 3 atomes de.carbone. Le contre ion est un halogène, de préférence le chlore.

Dans le cas des celluloses cationiques modifiées hydrophobes ou non, le groupement cationique est un groupement ammonium quaternaire portant trois radicaux, identiques ou non, choisis parmi l'hydrogène, un radical alkyle comprenant 1 à 10 atomes de carbone, plus particulièrement 1 à 6, de manière avantageuse 1 à 3 atomes de carbone. Le contre ion est un halogène, de préférence le chlore.

Parmi les dérivés cationiques de guar, on peut citer le guar hydroxypropyl trimonium chlorure (JAGUAR C13S, C14S, ou C17, JAGUAR Excel, JAGUAR C 2000 commercialisés par la société Rhodia Chimie) ou l'hydroxypropyl guar hydroxypropyl trimonium chlorure (JAGUAR C162 commercialisés par RHODIA).

Parmi les dérivés cationiques de cellulose, on pourra utiliser l'éther de poly(oxyéthanediyl-1,2) hydroxy-2 chlorure de triméthylammonium-3 propyl cellulose ou polyquatemium-10, comme le Polymer JR400 (dénomination INPI: PQ10) commercialisé par la société Amerchol.

Peuvent également être utilisés des dérivés non ioniques de polysaccharides, par exemple du guar hydroxypropyl.

Peuvent de même convenir, des polymères synthétiques, et plus particulièrement des homopolymères comme le polyméthacrylamidopropyl trimonium chlorure (Polycare 133 commercialisé par la société Rhodia Chimie).

Les polymères cationiques présentent plus particulièrement une masse molaire en poids d'au moins 2000 g/mol et plus préférentiellement comprise entre $2.10^4$ et $3.10^6$ g/mol, selon leur degré de polymérisation éventuelle. Les masses molaires en poids des polymères sont habituellement mesurées par exclusion de taille. Eventuellement, elles peuvent être mesurées directement par diffusion de la lumière ou à partir de la viscosité intrinsèque en utilisant un étalonnage selon : "Viscosity-Molecular weight relationship, intrinsic chain flexibility and dynamic solution properties of guar galactomannan" de G. Robinson, S.B. Ross Murphy, E.R. Morris, Carbohydrate Research 107, p.17-32, 1982.

Dans le cas des dérivés cationiques des polysaccharides, le degré d'hydroxyalkylation (substitution molaire ou MS) est de préférence compris entre 0 et 1,2. Toujours dans le cas de ces polymères, le degré de cationicité (degré de substitution ou DS) est plus particulièrement compris entre 0,01 et 0,6. C'est par exemple le cas des Jaguars C162 et C2000 commercialisés par la société Rhodia Chimie.

<u>Polyorganosiloxanes (silicone)</u>

La composition peut comprendre une silicone (huile silicone). On entend par silicone ou polyorganosiloxane tout composé organosiloxane, comprenant des groupes alkyle (par exemple méthyle) et/ou fonctionnalisés par des groupes différents des groupes alkyle.

Le polyorganosiloxane est avantageusement (dans les shampooings et après-shampooings en particulier) un polyorganosiloxane non volatil et non-hydrosoluble. Il présente avantageusement une viscosité comprise entre 1000 et 2000000 mPa.s, de préférence entre 5000 et 500000 mPa.s. Le polyorganosiloxane peut notamment être un polydiméthylorganosiloxanesiloxane ("PDMS", dénomination INCI: dimethicone), ou un polyorganosiloxane présentant des groupes amines (par exemple de l'Amodimethicone selon la dénomination INCI), ammonium quaternaire (par exemple les silicone quaternum 1 à 10 selon la dénomination INCI), hydroxyle (terminaux ou non), ou polyoxyalkylène, par exemple polyoxyde d'éthylène et/ou polyoxyde de propylène (en groupes terminaux, en bloc en sein d'une chaîne de PDMS, ou en greffons), ou plusieurs de ces groupes.

La quantité de polyorganosiloxane présent dans la composition peut typiquement être de 0, 1 % à 5% en poids, par exemple de 0,5 % à 1,5% ou 2%.

Le polyorganosiloxane (silicones) est de préférence présente dans la composition sous forme d'émulsion (gouttelettes liquides de silicone dispersée dans la phase aqueuse). L'émulsion peut notamment être une émulsion dont la taille moyenne des gouttelettes est supérieure ou égale à 2 $\mu$m, et/ou dont la taille moyenne des gouttelettes est supérieure ou comprise entre 0,15 $\mu$m et 2 $\mu$m, ou dont la taille moyenne des gouttelettes est inférieure ou égale à 0,15 $\mu$m.

Les gouttelettes de l'émulsion peuvent être de taille plus ou moins importante. On peut ainsi se référer à des microémulsions, à des mini-émulsions, ou à des macro-émulsions. Dans la présente demande, le terme «émulsion» couvre notamment tous ces types d'émulsions. Sans vouloir être lié à une quelconque théorie, on précise que les microémulsions sont généralement des systèmes thermodynamiquement stables, comprenant généralement d'importantes quantités d'agents d'émulsification. Les autres émulsions sont généralement des systèmes en état non-thermodynamiquement stable, conservant pendant un certain temps, en état métastable l'énergie mécanique fournie lors de

l'émulsification. Ces systèmes comprennent généralement des quantités moindres d'agents d'émulsification.

Les émulsions peuvent être obtenues par mélange du vecteur, de préférence aqueux, du polyorganosiloxane, et en général d'un agent d'émulsification, puis émulsification. On peut parler d'émulsification in situ.

Les compositions sous forme d'émulsion peuvent également être obtenues par mélange du vecteur, de préférence aqueux, avec une émulsion préalablement préparée de gouttelettes comprenant le polyorganosiloxane dans une phase externe, de préférence miscible avec le vecteur cosmétiquement acceptable, de préférence de même nature que ledit vecteur, de préférence un vecteur aqueux. On peut préférer ce mode de réalisation car il est simple à mettre en oeuvre. En outre ce mode de réalisation est particulièrement adapté pour la mise en oeuvre de compositions cosmétiques dans lesquelles le polyorganosiloxane est sous forme de microémulsion. On peut parler d'émulsification préalable.

Selon un mode particulier de réalisation, l'émulsion est une microémulsion, dont la taille des gouttelettes est inférieure à 0,15 $\mu$m. Dans ce mode de réalisation, la composition comprend de préférence une proportion supérieure à 10% en poids, de préférence au moins 15% en poids d'agent d'émulsification par rapport au poids de polyorganosiloxane.

La taille des gouttelettes de microémulsion peut être mesurée sur une émulsion préparée préalablement à son introduction dans la composition cosmétique, par diffusion de Lumière Dynamique (DQEL), par exemple comme décrit ci-après. L'appareillage utilisé est par exemple constitué d'un laser Spectra-Physics 2020, d'un corrélateur Brookhaven 2030 et de l'informatique associée. L'échantillon étant concentré, il est dilué dans l'eau désionisée et filtré à 0,22 $\mu$m, pour être en final à 2% en poids. Le diamètre obtenu est un diamètre apparent. Les mesures sont faites à 90° et 135° d'angle. Pour les mesures de taille, outre l'analyse classique par les cumulants, trois exploitations de la fonction d'autocorrélation sont utilisées (l'échantillonnage exponentiel ou EXPSAM décrit par le Pr. Pike, la méthode « Non Negatively Constrained Least Squares » ou NNLS et la méthode CONTIN décrite par le Pr. Provencher), qui donnent chacune une répartition de taille pondérée par l'intensité diffusée, et non pas par la masse ou le nombre. Il est tenu compte de l'indice de réfraction et de la viscosité de l'eau.

Selon un mode avantageux, la microémulsion est transparente. La microémulsion peut par exemple présenter une transmittance d'au moins 90%, de préférence d'au moins 95%, à une longueur d'onde de 600 nm, mesurée par exemple à l'aide d'un spectromètre Lambda 40 UV-Vis; à une concentration à 0.5% en poids dans l'eau. Dans ce cadre, la composition cosmétique peut avantageusement être transparente. Elle peut par exemple présenter une transmittance d'au moins 90%, de préférence d'au moins 95%, à une longueur d'onde de 600 nm, mesurée par exemple à l'aide d'un spectromètre Lambda 40 UV-Vis.

Selon un autre mode particulier de réalisation, l'émulsion est une émulsion dont la taille moyenne des gouttelettes est supérieure ou égale à 0,15 $\mu$m, par exemple supérieure à 0,5 $\mu$m, ou à 1 $\mu$m, ou à 2 $\mu$m, ou à 10 $\mu$m, ou à 20 $\mu$m, et de préférence inférieure à 100 $\mu$m. La taille des gouttelettes peut être mesurée sur une émulsion préparée préalablement à son introduction dans la composition cosmétique, ou directement sur la composition cosmétique diluée dans de l'eau, par microscopie optique et/ou granulomètrie laser (Horiba LA-910 laser scattering analyzer). Dans ce mode de réalisation, la composition comprend de préférence une proportion inférieure à 10% en poids d'agent d'émulsification, par rapport au poids de polyorganosiloxane.

Des agents d'émulsification utiles pour la réalisation d'émulsion de polyorganosiloxanes sont notamment les tensioactifs non ioniques, de préférence polyalcoxylés, par exemple choisis parmi, les alcools gras alcoxylés, les triglycérides alcoxylés, les acides gras alcoxylés, les esters de sorbitan alcoxylés, les amines grasses alcoxylées, les di (phényl-1 éthyl) phénols alcoxylés, les tri(phényl-1 éthyl) phénols alcoxylés, et les alkyls phénols alcoxylés, où le nombre de motifs alcoxy, plus particulièrement oxyéthyléne et/ou oxypropyléne, est tel que la valeur de HLB est supérieure ou égale à 10.

Parmi les dérivés de silicones solubles dans l'eau de la composition, on peut citer entre autres, les diméthicones copolyols (Mirasil DMCO commercialisée par la société Rhodia Chimie).

Pour ce qui a trait aux silicones se présentant sous forme de dispersions insolubles dans l'eau de la composition, on peut utiliser de manière convenable, des organopolysiloxanes non hydrosolubles et non volatils parmi lesquels on peut citer les huiles, gommes ou résines polyalkylsiloxanes, polyarylsiloxanes, polyalkylarylsiloxanes, ou leurs dérivés fonctionnalisés non hydrosolubles, ou leurs mélanges, non volatils.

Lesdits organopolysiloxanes sont considérés comme non hydrosolubles et non volatils, lorsque leur solubilité dans l'eau est inférieure à 50 g/litre et leur viscosité intrinsèque d'au moins 3000 mPa.s, à 25°C.

A titre d'exemples d'organopolysiloxanes ou silicones non hydrosolubles et non volatils, on peut citer des gommes silicones comme par exemple la gomme diphényl diméthicone commercialisée par la société Rhodia Chimie, et de préférence les polydiméthylsiloxanes présentant une viscosité au moins égale à $6.10^5$ mPa.s, à 25°C, et de façon encore plus préférentielle, ceux d'une viscosité supérieure à $2.\ 10^6$ mPa.s, à 25°C, tels que la Mirasil DM 500000® commercialisée par la société Rhodia Chimie.

Selon l'invention, l'organopolysiloxane ou silicone non hydrosoluble et non volatil se trouve sous une forme dispersée au sein de la composition cosmétique le renfermant.

Celui-ci se présente sous forme de particules ou de gouttelettes dont la taille peut être choisie en fonction de la nature de la composition cosmétique ou de la performance recherchée pour ladite composition. D'une manière générale, cette taille peut varier de 0,01 à 70 microns.

D'une manière préférentielle, cette taille est de l'ordre de 0,1 à 50 microns, tout particulièrement de l'ordre de 1 à 30 microns.

Pour faciliter leur mise en oeuvre, ces organopolysiloxanes peuvent être préalablement dispersés ou solubilisés dans des dérivés silicones de faible viscosité, volatils ou non, puis émulsionnés dans la composition cosmétique.

Parmi ces silicones de faible viscosité, on peut citer les silicones volatils cycliques et les polydiméthyl siloxanes de faible masse.

On pourra également utiliser des dérivés de silicones fonctionnalisés comme les dérivés aminés directement sous forme d'émulsions ou à partir d'une micro-émulsion préformée. Il peut s'agir de composés connus sous le terme de silicones aminées ou de silicones hydroxylées. On cite la Mirasil ADM-E (Amodiméthicone) commercialisée par la société Rhodia, et le dimethiconol.

A titre de polyorganosiloxanes pouvant être utilisés, on cite notamment:

- les polyorganosiloxanes comprenant des unités $-Si(CH_2)_2O-$ et des unités $-SiY(CH_2)O-$ où Y est un groupe $-(CH_2)_3-NH(CH_2)_2-NH_2$ ou $-(CH_2)_3-NH_2$
- les polyorganosiloxanes comprenant des unités $-Si(CH_2)_2O-$ et des unités terminales - $HO-Si(CH_2)_2O-$ et/ou des unités non terminales $-Si(CH_2)(OH)O-$
- les polyorganosiloxanes comprenant des unités $-Si(CH_2)_2O-$ et des unités
- $SiY(CH_2)O-$ où Y est $-L^X-Z^x-$Palc où $L^X$ est un groupe de liaison divalent, de préférence un groupe alkyle, $Z^X$ est une liaison covalente ou un groupe rotule divalent comprenant un hétéroatome, Palc est un groupe de formule $[OE]_s-[OP]_t-X'$, dans laquelle OE est un groupe de formule $-CH_2-CH_2-O-$, OP est un groupe de formule $-CH_2-CHCH_3-O-$ ou - $CHCH_3-CH_2-O-$, X' est un atome d'hydrogène ou un groupe de hydrocarboné, s est un nombre moyen supérieur à 1, et t est un nombre moyen supérieur ou égal à 0,
- les polyorganosiloxanes dont la chaîne comprend au moins un bloc comprenant des unités de formule des unités $-Si(CH_2)_2O-$ et au moins un bloc $-[OE]_s-[OP]_t-$,
- les polyorganosiloxanes comprenant des unités $-Si(CH_2)_2O-$ et/ou des unités $-Si(CH_2)RO-$ et/ou $-SiR_2O-$ et/ou $R-Si(CH_2)_2O-$ et/ou $H_3C-SiR_2O-$ et/ou $R-SiR_2O-$ où R, identique ou différent est un groupe alkyle différent d'un groupe méthyle, un groupe aryle, un groupe alkyle, un groupe alkyaryl, ou un groupe aralkyl.

Autres composés

Il est de même envisageable d'utiliser des huiles pouvant exercer des fonctions conditionnantes, protectrices, ou émollientes. De telles huiles sont généralement choisies parmi les alkylmonoglycérides, les alkyldiglycérides, les triglycérides comme les huiles extraites des plantes et des végétaux (huiles de palme, de coprah, de graine de coton, de soja, de tournesol, d'olive, de pépin de raisin, de sésame, d'arachide, de ricin...) ou les huiles d'origine animale (suif, huiles de poisson...), des dérivés de ces huiles comme les huiles hydrogénées, les dérivés de la lanoline, le petrolatum, les huiles minérales ou les huiles paraffiniques, le perhydrosqualane, le squalène, les diols comme le 1-2-dodécanediol, l'alcool cétylique, l'alcool stéarylique, l'alcool oléique, les esters gras comme le palmitate d'isopropyl, le cocoate d'éthyl-2-hexyl, le myristyl myristate, les esters de l'acide lactique, l'acide stéarique, l'acide béhénique, l'acide isostéarique.

On peut aussi incorporer dans la composition cosmétique, sous forme de dispersions ou de solutions, des agents bactéricides ou fongicides afin d'améliorer la désinfection de la peau, comme par exemple le triclosan ; des agents anti-pelliculaires, comme notamment le zinc pyrithione ou l'octopyrox ; des agents insecticides comme les pyréthroides naturels ou de synthèse.

Les compositions cosmétiques peuvent également contenir des agents pour la protection de la peau et/ou des cheveux contre les agressions du soleil et des rayons UV. Ainsi, les compositions peuvent comprendre des filtres solaires qui sont des composés chimiques absorbant fortement le rayonnement UV, comme les composés autorisés dans la directive européenne N° 76/768/CEE, ses annexes et les modifications ultérieures de cette directive.

Dans le cas où les divers éléments constitutifs de la composition cosmétique présentent une solubilité trop faible dans la composition ou lorsqu'ils se présentent sous forme solide à température ambiante, lesdits éléments constitutifs peuvent avantageusement être solubilisés dans un véhicule organique, comme des huiles minérales ou naturelles, des dérivés de silicones, des cires, ou bien encore encapsulés dans des matrices comme des polymères de type latex.

Les compositions cosmétiques faisant l'objet de l'invention peuvent également contenir des résines fixatives.

Ces résines fixatives, lorsqu'elles sont présentes, le sont généralement à des concentrations comprises entre 0,01 et 10%, préférentiellement entre 0,5 et 5%.

Les résines fixatives entrant dans les compositions cosmétiques sont plus particulièrement choisies parmi les

résines suivantes :

- les copolymères acrylate de méthyle / acrylamide, copolymères polyvinylméthyléther / anhydride maléique, copolymères acétate de vinyle / acide crotonique, copolymères octylacrylamide / acrylate de méthyle / butylaminoéthylméthacrylate, polyvinylpyrrolidones, copolymères polyvinylpyrrolidone / méthacrylate de méthyle, copolymères polyvinylpyrrolidone / acétate de vinyle, alcools polyvinyliques, copolymères alcool polyvinylique / acide crotonique, copolymères alcool polyvinylique / anhydride maléique, hydroxypropyl celluloses, hydroxypropyl guars, polystyrène sulfonates de sodium, terpolymères polyvinylpyrrolidone / éthyl méthacrylate/ acide méthacrylique, monométhyl éthers de poly(méthylvinyl éther / acide maléique), polyvinylacétates greffés sur des troncs polyoxyéthylènes (EP-A-219 048),
- les copolyesters dérivés d'acide, anhydride ou d'un diester téréphtalique et/ou isophtalique et/ou sulfoisophtalique et d'un diol, tels que :

  - les copolymères polyesters à base de motifs ethylène téréphtalate et/ou propylène téréphtalate et polyoxyéthylène téréphtalate, (US-A-3 959 230, US-A-3 893 929, US-A-4 116 896, US-A-4 702 857, US-A-4 770 666) ;
  - les oligomères polyesters sulfonés obtenus par sulfonation d'un oligomère dérivé de l'alcool allylique éthoxylé, du diméthyltéréphtalate et du 1,2 propylène diol (US-A-4 968 451);
  - les copolymères polyesters dérivés de diméthyltéréphtalate, d'acide isophtalique, de sulfoisophtalate de diméthyl et d'éthylène glycol (EP-A-540374)
  - les copolymères comprenant des unités polyesters dérivés de diméthyltéréphtalate, d'acide isophtalique, de sulfoisophtalate de diméthyl et d'éthylène glycol et d'unités polyorganosiloxanes (FR-A-2 728 915).
  - les oligomères polyesters sulfonés obtenus par condensation de l'acide isophtalique, du sulfosuccinate de diméthyle et de diéthylène glycol (FR-A-2 236 926)
  - les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896) ;
  - les polyesters-polyuréthanes obtenus par réaction d'un polyester obtenu à partir d'acide adipique et/ou d'acide téréphtalique et/ou d'acide sulfoisophtalique et d'un diol, sur un prépolymère à groupements isocyanates terminaux obtenus à partir d'un polyoxyéthylène glycol et d'un diisocyanate (FR-A-2 334 698) ;

- les monoamines ou polyamines éthoxylées, les polymères d'amines éthoxylées (US-A-4 597 898, EP-A-11 984).

De manière préférentielle, les résines fixatives sont choisies parmi les polyvinylpyrrolidone (PVP), copolymères de polyvinylpyrrolidone et de méthyl méthacrylate, copolymère de polyvinylpyrrolidone et d'acétate de vinyle (VA), copolymères polytéréphtale d'éthylène glycol / polyéthylène glycol, copolymères polytéréphtalate d'éthylène glycol / polyéthylène glycol / polyisophtalate sulfonate de sodium, et leurs mélanges.

Ces résines fixatives sont préférentiellement dispersées ou solubilisées dans le véhicule choisi.

Les compositions cosmétiques faisant l'objet de l'invention peuvent également contenir des dérivés polymériques exerçant une fonction protectrice.

Ces dérivés polymériques peuvent être présents en quantités de l'ordre de 0,01-10%, de préférence environ 0,1-5%, et tout particulièrement de l'ordre de 0,2-3% en poids.

Ces agents peuvent notamment être choisis parmi :

- les dérivés cellulosiques non ioniques tels que les hydroxyéthers de cellulose, la méthylcellulose, l'éthylcellulose, l'hydroxypropyl méthylcellulose, l'hydroxybutyl méthylcellulose ;
- les polyvinylesters greffés sur des troncs polyalkylénés tels que les polyvinylacétates greffés sur des troncs polyoxyéthylènes (EP-A-219 048) ;
- les alcools polyvinyliques.

Les compositions cosmétiques faisant l'objet de l'invention peuvent aussi comprendre des agents plastifiants.

Lesdits agents, s'ils sont présents, peuvent représenter entre 0,1 à 20% de la formulation de préférence de 1 à 15%.

Parmi les agents plastifiants particulièrement utiles, on peut citer les adipates, les phtalates, les isophtalates, les azélates, les stéarates, les silicones copolyols, les glycols, l'huile de ricin, ou leurs mélanges.

On peut aussi avantageusement ajouter à ces compositions des agents séquestrants des métaux, plus particulièrement ceux séquestrant le calcium comme les ions citrates.

On peut également incorporer aux compositions cosmétiques faisant l'objet de l'invention des agents humectants, parmi lesquels figurent, entre autres, le glycérol, le sorbitol, l'urée, le collagène, la gélatine, l'aloe vera, l'acide hyaluronique ou des solvants volatils hydrosolubles comme l'éthanol ou le propylène glycol dont les teneurs peuvent atteindre jusqu'à 60% en poids de la composition.

Pour diminuer encore l'irritation ou l'agression du cuir chevelu, on peut aussi ajouter des polymères hydrosolubles ou hydrodispersables comme le collagène ou certains dérivés non allergisants de protéines animales ou végétales (hydrolysats de protéines de blé par exemple), des hydrocolloïdes naturels (gomme de guar, de caroube, de tara, ...) ou issus de procédés de fermentation et les dérivés de ces polycarbohydrates comme les celluloses modifiées non ioniques comme par exemple l'hydroxyéthylcellulose, ou anionique comme la carboxyméthylcellulose ; les dérivés du guar ou de la caroube comme leurs dérivés non-ioniques (par exemple hydroxypropylguar) ou les dérivés anioniques (carboxyméthylguar et carboxyméthylhydroxypropylguar).

A ces composés, on peut ajouter en association, des poudres ou des particules minérales comme du carbonate de calcium, du bicarbonate de sodium, du dihydrogénophosphate de calcium, des oxydes minéraux sous forme de poudre ou sous forme colloïdale (particules de taille inférieure ou de l'ordre de un micromètre, parfois de quelque dizaines de nanomètres) comme du dioxyde de titane, de la silice, des sels d'aluminium utilisés généralement comme anti-transpirants, du kaolin, du talc, des argiles et leurs dérivés, etc..

Des agents conservateurs comme les méthyl, éthyl, propyl et butyl esters de l'acide p-hydroxybenzoïque, le benzoate de sodium, le GERMABEN® ou tout agent chimique évitant la prolifération des bactéries ou des moisissures et utilisé traditionnellement des les compositions cosmétiques, peuvent aussi être introduits dans les compositions aqueuses cosmétiques selon l'invention, généralement à hauteur de 0,01 à 3 % en poids.

La quantité de ces produits est habituellement ajustée pour éviter toute prolifération de bactéries, moisissures ou levures dans les compositions cosmétiques.

Alternativement à ces agents chimiques, on peut parfois utiliser des agents modifiant l'activité de l'eau et augmentant fortement la pression osmotique comme les carbohydrates ou des sels.

Pour protéger la peau et/ou les cheveux des agressions du soleil et des rayons UV, on peut ajouter aux compositions des filtres solaires organiques ou minéraux, par exemple des particules minérales comme l'oxyde de zinc, le dioxyde de titane ou les oxydes de cérium sous forme de poudre ou de particules colloïdales, seuls ou en mélange. Ces poudres peuvent éventuellement être traitées en surface pour augmenter l'efficacité de leur action anti-UV ou pour faciliter leur incorporation dans les formulations cosmétiques ou pour inhiber la photoréactivité de surface. Les filtres solaires organiques peuvent notamment être introduit dans le polyorganosiloxanes, s'il est présent dans la composition.

A ces ingrédients on peut ajouter, si nécessaire, et dans le but d'augmenter le confort lors de l'utilisation de la composition par le consommateur, un ou des parfums, des agents colorants parmi lesquels on peut citer les produits décrits dans l'annexe IV ("List of colouring agents allowed for use in cosmetic products") de la directive européenne n° 76/768/CEE du 27 juillet 1976 dite directive cosmétique, et/ou des agents opacifiants comme des pigments.

Bien que cela ne soit pas obligatoire, la composition peut aussi contenir des polymères viscosants ou gélifiants de façon à ajuster la texture de la composition, comme les polyacrylates réticulés (Carbopol commercialisés par Goodrich), déjà mentionnés ci-dessus, les dérivés non cationiques de la cellulose comme l'hydroxypropylcellulose, la carboxyméthylcellulose, les guars et leurs dérivés non ioniques, la gomme xanthane et ses dérivés, utilisés seuls ou en association, ou les mêmes composés, généralement sous la forme de polymères hydrosolubles modifiés par des groupements hydrophobes liés de manière covalente au squelette polymère comme décrit dans le brevet WO 92/16187 et/ou de l'eau pour amener le total des constituants de la formulation à 100 %.

Les compositions cosmétiques faisant l'objet de l'invention peuvent également contenir des agents dispersants polymériques en quantité de l'ordre de 0,1-7% en poids, pour contrôler la dureté en calcium et magnésium, agents tels que :

- les sels hydrosolubles d'acides polycarboxyliques de masse moléculaire en poids de l'ordre de 2000 à 100000 g/mol, obtenus par polymérisation ou copolymérisation d'acides carboxyliques éthyléniquement insaturés tels que l'acide acrylique, l'acide ou l'anhydride maléique, l'acide fumarique, l'acide itaconique, l'acide aconitique, l'acide mésaconique, l'acide citraconique, l'acide méthylènemalonique, et tout particulièrement les polyacrylates de masse moléculaire en poids de l'ordre de 2 000 à 10 000 g/mol (US-A-3 308 067), les copolymères d'acide acrylique et d'anhydride maléique de masse moléculaire en poids de l'ordre de 5000 à 75 000 g/mol (EP-A-66 915) ;
- les polyéthylèneglycols de masse moléculaire en poids de l'ordre de 1000 à 50000 g/mol.

<u>Composition pour les soins ménagers, industriels ou institutionnels</u>

Selon une mode de réalisation particulier la composition est une composition (ou "formulation") pour les soins ménagers, industriels ou institutionnels. Il peut s'agir notamment d'une composition nettoyante ou rinçante.

La composition peut être destinée au traitement de surfaces dures industrielles, domestiques ou de collectivité, notamment de type céramique, carrelage, vitre, métal, mélamine, formica ou plastique, visant à conférer à celles-ci

notamment des propriétés rémanentes antidéposition et/ou antiadhésion des salissures ; elle peut en outre apporter à celles-ci des propriétés d'antistatisme, de brillance, des propriétés antidérapantes.

La composition destinée au traitement d'une surface dure, composition est apte à conférer à celle-ci des propriétés rémanentes antidéposition et/ou antiadhésion des salissures, de manière à éviter la présence ultérieure de traces dues en particulier :

- au séchage des gouttes d'eau déposées sur ladite surface (par exemple dépôt de sels minéraux).
- à l'accrochage de particules minérales ou organiques présentes dans l'air ambiant (cas du nettoyage de gratte-ciels) ou déposées par contact (cas du nettoyage des sols, des toilettes ...)
- au dépôt par éclaboussure de composés organiques gras (graisses de cuisine)
- au dépôt de savons et de leur sels métalliques
- au dépôt de composés d'origine végétales de type hydrocolloides ou polysaccharides.

Par « propriétés rémanentes antidéposition et/ou antiadhésion », on entend que la surface traitée conserve ces propriétés au cours du temps, y compris après des contacts ultérieurs avec une salissure (par exemple eau de pluie, eau du réseau de distribution eau de rinçage additionnée ou non de produits de rinçage, éclaboussures grasses, savons...). Cette propriété de rémanence peut être observée au delà d'une dizaine de cycles de rinçage, voire dans certains cas particuliers où les rinçages sont nombreux (cas des toilettes par exemple), au delà de 100 cycles de rinçage.

L'expression ci-dessus de « conférer à la surface ainsi traitée des propriétés antidéposition » signifie plus particulièrement que la surface traitée, mise en contact avec une salissure dans un milieu majoritairement aqueux, n'aura pas tendance à « capter » ladite salissure, ce qui diminue ainsi significativement le dépôt de la salissure sur la surface.

L'expression ci-dessus de « conférer à la surface ainsi traitée des propriétés antiadhésion » signifie plus particulièrement que la surface traitée n'est susceptible d'interagir que très faiblement avec la salissure qui s'y est déposée, ce qui permet un enlèvement facile des salissures de la surface traitée salie ; en effet lors du séchage de la salissure mise au contact de la surface traitée, les liaisons développées entre la salissure et la surface sont très faibles ; ainsi, casser ces liaisons demande moins d'énergie (donc d'efforts) lors de l'opération de nettoyage.

Lorsqu'il est dit que la présence du copolymère permet « d'améliorer la capacité nettoyante » d'une formulation, cela signifie que pour une même quantité de formulation nettoyante (notamment une formulation de lavage de la vaisselle à la main), la formulation contenant le copolymère permet de nettoyer un plus grand nombre d'objets souillés qu'une formulation qui en est exempte.

En outre le dépôt sur une surface dure du copolymère permet d'apporter à cette surface des propriétés d'antistatisme ; cette propriété est particulièrement intéressante dans le cas de surfaces synthétiques.

La présence du copolymère dans les formulations de traitement d'une surface dure permet de rendre la surface hydrophile ou d'améliorer son hydrophilie.

La propriété d'hydrophilisation de la surface permet de plus de réduire la formation de buée sur la surface ; ce bénéfice peut être exploité dans les formules de nettoyage pour les vitres et les miroirs, en particulier en salles de bain. De plus, la vitesse de séchage de la surface, immédiatement après son traitement par l'application du polymère mais également après des contacts ultérieurs et répétés avec un milieu aqueux est améliorée de manière très significative.

Le terme « surfaces dures » est à prendre au sens large ; il s'agit de surfaces non-textiles, qui peuvent être aussi bien ménagères, de collectivité, qu'industrielles. Elles peuvent être en un matériau quelconque, notamment du type :

- céramique (surfaces telles que lavabo, baignoires, carrelages muraux ou au sol, cuvettes des toilettes...)
- verre (surfaces telles que vitres intérieures et extérieures de bâtiments ou de véhicules, miroirs,
- métal (surfaces telles que parois internes ou externes de réacteurs, lames, panneaux, tuyaux....)
- résines synthétiques (par exemple carrosseries ou surfaces intérieures de véhicules motorisés (voitures, camions, bus, trains, avions ...) surfaces en mélamine ou formica pour l'intérieur de bureaux, cuisines, ...))
- matières plastiques (par exemple polychlorure de vinyle, polyamide, pour l'intérieur des véhicules, voitures notamment)

Les « surfaces dures », selon l'invention, sont des surfaces peu poreuses et non fibrillaires ; elles sont ainsi à distinguer des surfaces textiles (tissus, moquettes, vêtements ... en matériaux naturels, artificiels ou synthétiques).

La composition selon l'invention, susceptible d'apporter aux surfaces dures à traiter des propriétés antidéposition et/ou antiadhésion des salissures, peut être : ➢ Une composition à usage ménager; elle peut être universelle ou peut être plus spécifique, comme une composition pour le nettoyage ou le rinçage

- de salle de bain ; ladite composition empêche notamment le dépôt des sels de savon autour des baignoires et sur

les lavabos, prévient la croissance et/ou le dépôt de cristaux de calcaire sur ces surfaces, et retarde l'apparition de tâches de savon ultérieures.

- de cuisine ; ladite composition permet d'améliorer le nettoyage des plans de travail lorsque ceux-ci sont souillés par des salissures grasses insaturées susceptibles de réticuler dans le temps ; les tâches grasses partent à l'eau sans frotter.
- des sols (en linoleum, carrelage ou ciment) ; ladite composition permet d'améliorer l'enlèvement des poussières, des salissures de types argilo-calcaires (terre, sable, boue...) ; les tâches sur le sol peuvent être nettoyées sans effort par simple balayage, sans brossage ; en outre ladite composition apporte des propriétés antidérapantes.
- des toilettes ; ladite composition permet d'éviter l'adhésion de traces d'excréments sur la surface ; le seul flux de la chasse d'eau est suffisant pour éliminer ces traces ; l'utilisation dune brosse est inutile.
- des vitres ou miroirs ; ladite composition permet d'éviter le dépôt de salissures particulaires minérales ou organiques sur la surface.
- de la vaisselle, à la main ou à l'aide d'une machine automatique ; ladite composition permet, dans le cas du lavage à la main, de faciliter l'enlèvement des tâches résiduelles d'aliments séchés, et de laver un plus grand nombre de couverts ou ustensiles avec un même volume de bain ; la surface des couverts et ustensiles encore mouillés n'est plus glissante et ainsi n'échappe pas des mains de l'utilisateur ; il a également été constaté un effet « squeaky clean », à savoir que la surface « crisse » sous l'effet d'un frottement avec le doigt. Dans le cas du lavage ou du rinçage en lave-vaisselle, ladite composition permet l'anti-redéposition des salissures alimentaires et des sels minéraux insolubles du calcium, et apporte de la brillance aux ustensiles et couverts ; la composition permet également de ne plus avoir à « prélaver » les couverts ou ustensiles avant leur introduction dans le lave-vaisselle.

➢ Une composition à usage industriel ou de collectivité ; elle peut être universelle ou plus spécifique, comme une composition pour le nettoyage

- des réacteurs, des lames en acier, des éviers, des cuves.
- de la vaisselle
- des surfaces extérieures ou intérieures des bâtiments
- des vitres des bâtiments et immeubles
- des bouteilles

La composition selon l'invention peut se présenter sous une forme quelconque et peut être utilisée de multiples façons.

Ainsi, elle peut être sous la forme

- d'un liquide gélifié ou non, à déposer tel quel, notamment par pulvérisation,
  - directement sur les surfaces à nettoyer ou rincer, ou
  - sur une éponge ou un autre support (article en cellulose par exemple, tissé ou non-tissé) avant d'être appliqué sur la surface à traiter
- d'un liquide gélifié ou non, à diluer dans de l'eau (éventuellement additionnée d'un autre solvant) avant d'être appliqué sur la surface à traiter
- d'un liquide gélifié ou non, emprisonné dans un sachet hydrosoluble
- d'une mousse
- d'un aérosol
- d'un liquide absorbé sur un support absorbant en un article tissé ou non-tissé notamment (lingette)
- d'un solide, tablette notamment, éventuellement emprisonnée dans un sachet hydrosoluble, ladite composition pouvant représenter tout ou partie de la tablette.

Pour une bonne réalisation de l'invention, le copolymère est présent dans la composition faisant l'objet de l'invention en quantité efficace pour apporter auxdites surfaces des propriétés antidéposition et/ou antiadhésion des salissures susceptibles de se déposer sur lesdites surfaces.

Ladite composition faisant l'objet de l'invention peut contenir, selon son application, de 0,001 à 10% de son poids du copolymère.

Le pH de la composition ou le pH d'utilisation de la composition selon l'invention, peut varier, selon les applications et les surfaces à traiter, de 1 à 14, voire même de 0,5 à 14.

Les pH extrêmes sont classiques dans les applications de type nettoyage industriel ou de collectivité. Dans le domaine des applications ménagères, les pH vont plutôt de 1 à 13 suivant les applications.

Ladite composition peut être mise en oeuvre pour le nettoyage ou le rinçage des surfaces dures, en quantité telle que, après rinçage éventuel et séchage, la quantité du copolymère déposée sur la surface soit de 0,0001 à 10 mg/m$^2$, de préférence de 0,001 à 5 mg/m$^2$ de surface traitée.

La composition selon l'invention peut comprendre en outre au moins un agent tensioactif. Celui-ci peut être non-ionique, anionique, amphotère, zwitterionique ou cationique. Des tensioactifs utiles ont été mentionnés ci-dessus pour les compositions cosmétiques.

Les agents tensioactifs peuvent représenter de 0,005 à 60 %, notamment de 0,5 à 40% du poids de la composition de l'invention, ce en fonction de la nature du ou des agent(s) tensioactif(s) et de la destination de la composition nettoyante.

Avantageusement, le rapport pondéral copolymère / agent(s) tensioactif(s), est compris entre 1/1 et 1/1000, avantageusement 1/2 et 1/200.

La composition selon l'invention, peut en outre comprendre au moins un additif autre, notamment choisi parmi les additifs usuels présents dans les compositions de nettoyage ou de rinçage des surfaces dures.

On peut notamment citer :

♦ des <u>agents chélatants</u>, notamment du type phosphonates organiques et aminophosphonates hydrosolubles tels que les

- éthane 1-hydroxy-1, 1-diphosphonates,
- aminotri(méthylène diphosphonate)
- vinyldiphosphonates
- sels des oligomères ou polymères de l'acide vinylphosphonique ou vinyldiphosphonique
- sels de co-oligomères ou copolymères statistiques de l'acide vinylphosphonique ou vinyldiphosphonique et de l'acide acrylique et/ou de l'anhydride maleïque et/ou de l'acide vinylsulfonique et/ou de l'acrylamidométhylpropane sulfonique
- sels d'acides polycarboxyliques phosphonés
- polyacrylates à terminaison(s) phosphonate(s)
- sels de cotélomères de l'acide vinylphosphonique ou vinyldiphosphonique et d'acide acrylique

comme ceux de la gamme BRIQUES® ou MIRAPOL A300 ou 400 de RHODIA (à raison de 0 à 10 %, de préférence de 0 à 5% du poids total de composition nettoyante);

♦ des <u>agents séquestrants ou antitartre</u> comme

- les acides polycarboxyliques ou leurs sels hydrosolubles et les sels hydrosolubles de polymères ou de copolymères carboxyliques tels que les
  - éthers polycarboxylates ou hydroxypolycarboxylates
  - acides polyacétiques ou leurs sels (acide nitriloacétique, acide N,N-dicarboxyméthyl-2-aminopentane dioïque, acide éthylènediamine tétraacétique, acide diéthylènetriamine pentaacétique, éthylènediaminetetraacétates, nitrilotriacétates, N-(2 hydroxyéthyl)-nitrilodiacétates),
  - sels d'acides alkyl $C_5$-$C_{20}$ succiniques
  - esters polyacétals carboxyliques
  - sels d'acides polyaspartiques ou polyglutamiques
  - acide citrique, acide adipique, acide gluconique ou acide tartrique ou leurs sels
- des copolymères d'acide acrylique et d'anhydride maléïque ou des homopolymères d'acide acrylique, tels que le Rhodoline DP 226 35 de Rhodia et le Sokalan CP5 de BASF (à raison de 0 à 10 %, du poids total de ladite composition nettoyante) ;
- des polyvinylstyrènes sulfonés ou leurs copolymères avec l'acide acrylique, méthacrylique ...
  (à raison de 0 à 10 %, du poids total de composition nettoyante);

♦ des <u>"builders"</u> (adjuvants de détergence améliorant les propriétés de surface des tensioactifs) minéraux du type :

- polyphosphates de métaux alcalins, d'ammonium ou d'alcanolamines tels que le RHODIAPHOS HD7 commercialisé par la société RHODIA, (à raison de 0 à 70 % du poids total de composition nettoyante) ;
- pyrophosphates de métaux alcalins
- silicates de métaux alcalins, de rapport $SiO_2/M_2O$ pouvant aller de 1 à 4, de préférence de 1,5 à 3,5, tout particulièrement de 1,7 à 2,8 ; il peut s'agir de silicates amorphes ou de silicates lamellaires comme les phases

alpha-, beta-, gamma- et delta- de $Na_2Si_2O_5$, commercialisées sous les références NaSKS-5, NaSKS-7, NaSKS-11 et NaSKS-6 par CLARIANT ;

- borates, carbonates, bicarbonates, sesquicarbonates alcalins ou alcalino-terreux (en quantité pouvant aller jusqu'à 50 % environ du poids total de ladite composition nettoyante);
- cogranulés de silicates hydratés de métaux alcalins de rapport $SiO_2/M_2O$ pouvant aller de 1,5 à 3,5, et de carbonates de métaux alcalins (sodium ou de potassium) ; on peut citer en particulier les cogranulés dans lesquels la teneur pondérale en eau associée au silicate par rapport au silicate sec est d'au moins 33/100, le rapport pondéral du silicate au carbonate pouvant aller de 5/95 à 45/55, de préférence de 15/85 à 35/65, tels que décrits dans EP-A-488 868 et EP-A-561 656, comme le NABION 15 commercialisé par la société RHODIA ; (la quantité totale de "builders" pouvant représenter jusqu'à 90% du poids total de ladite composition nettoyante ou rinçante) ;

♦ des agents de blanchiment du type perborates, percarbonates associés ou non à des activateurs de blanchiment acétylés comme la N, N, N', N'-tétraacétyl-éthylènediamine (TAED) ou des produits chlorés du type chloroisocyanurates, ou des produits chlorés du type hypochlorites de métaux alcalins, ou de l'eau oxygénée (à raison de 0 à 30 % du poids total de ladite composition nettoyante)

♦ des charges du type sulfate de sodium, chlorure de sodium, carbonate de sodium ou de calcium, kaolin, silice, à raison de 0 à 50 % du poids total de ladite composition;

♦ des catalyseurs de blanchiment contenant un métal de transition, les complexes de fer, manganèse et cobalt notamment, comme ceux du type $[Mn^{IV}_2(\mu\text{-}O)_3(Me_3TACN)_2](PF_6)_2$, $[Fe^{II}(MeN_4py)(MeCN)](ClO_4)_2$, $[(Co^{III})(NH_3)_5(OAc)](OAc)_2$, décrits dans US-A-4,728,455 , 5,114,606, 5,280,117 , EP-A-909 809, US-A-5,559,261 , WO 96/23859, 96/23860 et 96/23861 (à raison de 0 à 5 % du poids total de ladite composition nettoyante)

♦ des agents influant sur le pH de la composition, solubles dans le milieu nettoyant ou rinçant, notamment

- des additifs alcalinisants phosphates de métaux alcalins, carbonates, perborates, hydroxydes de métaux alcalins) ou
- des additifs acidifiants éventuellement nettoyants comme les acides minéraux (acide phosphoriques, polyphosphoriques, sulfamique, chlorhydrique, fluorhydrique, sulfurique, nitrique, chromique), les acides carboxyliques ou polycarboxyliques (acide acétique, hydroxyacétique, adipique, citrique, formique, fumarique, gluconique, glutarique, glycolique, malique, maléique, lactique, malonique, oxalique, succinique et tartrique) ou des sels d'acides comme le bisulfate de sodium, bicarbonates et sesquicarbonates de métaux alcalins.

♦ des polymères utilisés pour contrôler la viscosité du mélange et/ou la stabilité des mousses formées à l'utilisation, comme les dérivées de cellulose ou de guar (carboxyméthylcellulose, hydroxyéthylcellulose, hydroxypropylguar, carboxyméthylguar, carboxyméthylhydroxypropyl-guar...), la gomme xanthane, le succinoglycane (RHEOZAN® commercialisé par RHODIA), la gomme caroube, les carragénanes (à raison de 0 à 2 % du poids total de ladite composition nettoyante)

♦ des agents hydrotropes, comme les alcools courts en $C_2$-$C_8$, en particulier l'éthanol, les diols et glycols comme le diéthylène glycol, dipropylène-glycol, le xylène sulfonate de sodium, le naptalène sulfonate de sodium (à raison de 0 à 10g pour 100g de ladite composition nettoyante)

♦ des agents hydratants ou humectants pour la peau comme le glycérol, l'urée ou des agents protecteurs de la peau, comme les protéines ou hydrolysats de protéines, les huiles végétales comme l'huile de soja, les polymères cationiques comme les dérivés cationiques du guar (JAGUAR C13S®, JAGUAR C162®, HICARE 1000® commercialisés par la société RHODIA, (à raison de 0 à 40% du poids total de ladite composition nettoyante)

♦ des biocides ou désinfectants comme

- les biocides cationiques, par exemple

  * les sels de monoammonium quaternaire tels que

    - les chlorures de coco-alkyl benzyl diméthylammonium, de $C_{12}$-$C_{14}$ alkyl benzyl diméthylammonium, de coco-alkyl dichlorobenzyl diméthylammonium, de tetradecyl benzyl diméthylammonium, de didécyl diméthylammonium, de dioctyl diméthylammonium
    - les bromures de myristyl triméthylammonium, de cétyl triméthylammonium

  * les sels d'amines hétérocycliques monoquaternaires tels que les chlorures de laurylpyridinium, de cétylpyridinium, de $C_{12}$-$C_{14}$ alkyl benzyl imidazolium
  * les sels d'alkyl gras triphényl phosphonium comme le bromure de myristyl triphényl phosphonium

* les biocides polymères, comme ceux dérivés de la réaction

- de l'épichlorhydrine et de la diméthylamine ou de la diéthylamine
- de l'épichlorhydrine et de l'imidazole
- du 1,3-dichoro-2-propanol et de la diméthylamine
- du 1,3-dichoro-2-propanol et du 1,3-bis-diméthylamino-2-propanol
- du dichlorure d'éthylène et du 1,3-bis-diméthylamino-2-propanol
- du bis (2-chloroéthyl)ether et de la N,N'-bis(diméthylaminopropyl) urée ou thiourée
- les chlorhydrates de polymère de biguanidine, comme le VANTOCIL IB

• les biocides amphotères comme les dérivés de N-(N'-$C_8$-$C_{18}$alkyl-3-aminopropyl)-glycine, de N-(N'-(N''-$C_8$-$C_{18}$alkyl-2-aminoéthyl)-2-aminoéthyl)-glycine, de N,N-bis(N'-$C_8$-$C_{18}$alkyl-2-aminoéthyl)-glycine, tels que le (dodécyl) (aminopropyl) glycine, le (dodécyl) (diéthylènediamine) glycine
• les amines comme la N-(3-aminopropyl)-N-dodecyl-1,3-propanediamine
• les biocides halogénés comme les iodophores et sels d'hypochlorites, tels que le dichloroisocyanurate de sodium
• les biocides Phénoliques comme le phénol, le résorcinol, les crésols, l'acide salicylique
• les biocides hydrophobes comme

- le parachlorométaxylenol, le dichlorométaxylenol
- le 4-chloro-m-crésol
- le résorcinol monoacétate
- les mono- ou poly-alkyl ou aryl phénols, crésols ou résorcinols, comme l'o-phenyl-phénol, le p-tert-butyl-phénol, le 6-n-amyl-m-crésol,
- les alkyl et/ou aryl chloro ou bromophénols, comme l'o-benzyl-p-chlorophénol
- les diphényléthers halogénés, comme le 2',4,4'-trichloro-2-hydroxy-diphényl éther (triclosan), le 2,2'-dihydroxy-5,5'-dibromo-diphényl éther.
- le chlorophénésine (éther p-chloro-phénylglycérique).
  à raison de 0 à 5% du poids total de ladite composition nettoyante.

♦ des solvants avant une bonne activité nettoyante ou dégraissante, comme

- les alkylbenzenes de type octyl benzene,
- les oléfines ayant un point d'ébullition d'au moins 100°C, comme les alpha-olefines, preferentiellement le 1-decene or 1-dodecene
- les éthers de glycol de formule générale, R1 O(R2O)mH où R1 est un groupe alkyle présentant de 3 à 8 carbones et chaque R2 est soit un ethylene ou propylene et m est un nombre qui varie de 1 à 3 ; on peut citer les monopropyleneglycol monopropyl ether, dipropyleneglycol monobutyl ether, monopropyleneglycol monobutyl ether, diethyleneglycol monohexyl ether, monoethyleneglycol monohexyl ether, monoethyleneglycol monobutyl ether et leurs mélanges.
- les diols présentant de 6 à 16 atomes de carbone dans leur structure moléculaire ; les diols sont particulièrement intéressants car en plus de leur propriétés dégraissantes, ils peuvent aider à éliminer les sels de calcium (savons) ; les diols contenant de 8 à 12 atomes de carbone sont préférés, tout préférentiellement le 2,2,4-trimethyl-1,3-pentanediol.
- d'autres solvants tels que l'huile de pin, les terpenes d'orange, l'alcool benzylique, le n-hexanol, les esters phatliques alcools possédant 1 à 4 atomes de carbone, le butoxy propanol, le Butyl Carbitol et le 1(2-n-butoxy-1-methylethoxy)propane-2-ol aussi appelé butoxy propoxy propanol ou dipropylene glycol monobutyl ether, le diglycol hexyl (Hexyl Carbitol), butyl triglycol, les diols comme le 2,2,4-trimethyl-1,3-pentanediol, et leurs mélanges.

(à raison de 0 à 30% du poids total de ladite composition nettoyante)
♦ des nettoyants industriels comme les solutions de sels alcalins du type phosphates, carbonates, silicates ... de sodium, potassium, (à raison de 0 à 50% du poids total de ladite composition nettoyante)
♦ les solvants organiques hydrosolubles peu nettoyants comme le methanol, l'ethanol, l'isopropanol, l'ethylene glycol, le propylene glycol, et leur mélanges, (à raison de 0 à 40% du poids total de ladite composition nettoyante)
♦ des cosolvants comme la monoéthanolamide et/ou les béta-aminoalcanols, particulièrement intéressants dans les compositions de pH supérieur à 11, tout particulièrement supérieur à 11,7 , car ils aident à réduire la formation de films et de traces sur les surfaces dures (ils peuvent être mis en oeuvre à raison de 0,05 à 5% du poids de la composition nettoyante) ; des systèmes solvants comprenant de la monoéthanolamide et/ou des béta-aminoalcanols

sont décrits dans US 5,108,660.

♦ des agents antimousses comme les savons notamment. Les savons sont des sels alcalins d'acides gras, notamment les sels de sodium, potassium, ammonium et d'alcanol ammonium d'acides gras supérieurs contenant environ de 8 à 24 atomes de carbone, et de préférence d'environ 10 à environ 20 atomes de carbone ; on peut notamment citer les sels de mono-, di- et triéthanolamine de sodium et de potassium ou de mélanges d'acides gras dérivés de l'huile de coprah et d'huile de noix broyée. La quantité de savon peut être d'au moins 0,005 % en poids, de préférence de 0,5 % à 2 % en poids par rapport au poids total de la composition. Des exemples supplémentaires de matériaux de régulation de la mousse sont les solvants organiques, la silice hydrophobe, l'huile de silicone et les hydrocarbures.

♦ des abrasifs, comme la silice, le carbonate de calcium

♦ des additifs divers tels que des enzymes, des parfums, des colorants, des agents inhibiteurs de corrosion des métaux, des conservateurs, des brillanteurs optiques, des agents opacifiants ou perlescents ...

Le pH de la composition faisant l'objet de l'invention ou le pH d'utilisation de ladite composition peut aller de 0,5 à 14, de préférence de 1 à 14.

Les compositions de type alcalin, de pH supérieur ou égal à 7,5, de préférence supérieur à 8,5 pour les applications ménagères (tout particulièrement de pH de 8,5 à 12, notamment de 8,5 à 11,5) sont particulièrement utiles pour l'enlèvement de salissures grasses et sont particulièrement bien adaptées au nettoyage de cuisine. Elles peuvent comprendre de 0,001 à 5%, de préférence de 0,005 à 2% de leur poids du copolymère.

Les compositions alcalines comprennent généralement, à côté du copolymère, au moins un additif choisi parmi

- un agent séquestrant ou antitartre (en quantité allant de 0 à 40%, de préférence de 1 à 40%, plus préférentiellement de 2 à 30% et tout particulièrement de 5 à 20% du poids de la composition)
- un biocide ou désinfectant cationique, notamment de type ammonium quaternaire, comme les chlorures de N-alkyl benzyl dimethyl ammonium, chlorure de N-alkyl dimethyl ethylbenzyl ammonium, halogénure de N-didecydimethylammonium, et chlorure de di- N-alkyl dimethyl ammonium (en quantité pouvant aller de 0 à 60%, de préférence de 0 à 40%, plus préférentiellement de 0 à 15% et tout particulièrement de 0 à 5% du poids de la composition)
- au moins un agent tensioactif non-ionique, amphotère, zwitterionique, ou anionique ou leur mélange; lorsqu'un agent tensioactif cationique est présent, ladite composition comprend en outre préférentiellement un agent tensioactif amphotère et/ou non-ionique (la quantité totale d'agents tensioactifs peut aller de 0 à 80%, de préférence de 0 à 50% , tout particulièrement de 0 à 35% du poids de la composition)
- si nécessaire, un agent de régulation de pH, en une quantité permettant d'atteindre, éventuellement après dilution ou mise en solution de la composition, un pH d'utilisation allant de 7,5 à 13 ; l'agent de régulation de pH peut notamment être un système tampon comprenant de la monoethanolamine et/ou un beta-aminoalkanol et potentiellement mais préférentiellement des matériaux alcalins « co-tampon » du groupe de l'ammoniaque, des C2-C4 alkanolamines, des hydroxydes d'alcalins, silicates, borates, carbonates, bicarbonates et leur mélanges. Les cotampons préférés sont les hydroxydes alcalins.
- de 0,5 à 98%, de préférence de 25 à 95%, tout particulièrement de 45 à 90% en poids d'eau
- un solvant organique nettoyant ou dégraissant, en quantité pouvant représenter de 0 à 60%, de préférence de 1 à 45%, tout particulièrement de 2 à 15% du poids de ladite composition
- un co-solvant comme la monoethanolamine et/ou les beta-aminoalkanols, en quantité pouvant représenter de 0 à 10%, de préférence de 0,05 à 10%, tout particulièrement de 0,05 à 5% du poids de ladite composition
- un solvant organique hydrosoluble peu nettoyant, en quantité pouvant représenter de 0 à 25%, de préférence de 1 à 20 %, tout particulièrement de 2 à 15% du poids de ladite composition
- éventuellement un agent de blanchiment, un parfum ou d'autres additifs usuels.

Lesdites compositions alcalines peuvent se présenter sous la forme d'une formule prête à l'emploi ou bien d'une formule sèche ou concentrée à diluer dans l'eau notamment, avant emploi ; elles peuvent être diluées de 1 à 10 000 fois, de préférence de 1 à 1000 fois avant emploi.

Avantageusement, une formulation pour le nettoyage des cuisines, comprend :

- de 0,001 à 1 % en poids du copolymère
- de 1 à 10 % en poids de solvant hydrosoluble, l'isopropanol notamment
- de 1 à 5 % en poids de solvant nettoyant ou dégraissant, le butoxypropanol notamment
- de 0,1 à 2 % en poids de monoéthanolamine
- de 0 à 5 % en poids d'au moins un agent tensioactif non cationique, de préférence amphotère ou non-ionique,
- de 0 à 1 % en poids d'au moins un agent tensioactif cationique à propriété désinfectante (notamment mélange de n-alkyl dimethyl ethylbenzyl ammonium chloride et n-alkyl dimethyl benzyl ammonium chloride),
  la quantité totale d'agent(s) tensioactif(s) représentant de 1 à 50 % en poids

- de 0 à 2 % en poids d'un diacide carboxylique comme agent antitartre
- de 0 à 5 % d'un agent de blanchiment
- et de 70 à 98 % en poids d'eau.

Le pH d'une telle formulation est de préférence de 7,5 à 13, plus préférentiellement de 8 à 12.

Les compositions de type acide, de pH inférieur à 5, sont particulièrement utiles pour l'enlèvement de salissures de type minéral ; elles sont particulièrement bien adaptées au nettoyage de cuvettes de toilettes.

Elles peuvent comprendre de 0,001 à 5 %, de préférence de 0,01 à 2 % de leur poids du copolymère.

Les compositions acides comprennent généralement, à côté du copolymère,

- un agent acide minéral ou organique (en quantité allant de 0,1 à 40%, de préférence de 0,5 à 20% et plus préférentiellement de 0,5 à 15% du poids de la composition)
- au moins un agent tensioactif non-ionique, amphotère, zwitterionique, ou anionique ou leur mélange; (la quantité totale d'agents tensioactifs peut aller de 0,5 à 20%, de préférence de 0,5 à 10 % du poids de la composition)
- éventuellement un biocide ou désinfectant cationique, notamment de type ammonium quaternaire, comme les chlorures de N-alkyl benzyl dimethyl ammonium, chlorure de N-alkyl dimethyl ethylbenzyl ammonium, halogénure de N-didecydimethylammonium, et chlorure de di- N-alkyl dimethyl ammonium (en quantité pouvant aller de 0,01 à 2% de préférence de 0,1 à 1% du poids de la composition)
- éventuellement un agent épaississant (en quantité allant de 0,1 à 3%, du poids de la composition)
- éventuellement un agent de blanchiment (en quantité allant de 1 à 10%, du poids de la composition)
- de 0,5 à 99 %, de préférence de 50 à 98 % en poids d'eau
- un solvant, comme le glycol ou un alcool, (en quantité pouvant aller de 0 à 10% de préférence de 1 à 5% du poids de la composition)
- éventuellement un parfum, un conservateur, un abrasif ou d'autres additifs usuels.

Lesdites compositions acides se présentent de préférence sous la forme d'une formule prête à l'emploi.

Avantageusement, une formulation pour le nettoyage des cuvettes de toilettes, comprend :

- de 0,05 à 5%, de préférence de 0,01 à 2% en poids du copolymère
- une quantité d'agent acide nettoyant telle que le pH final de la composition soit de 0,5 à 4, de préférence de 1 à 4 ; cette quantité est généralement de 0,1 à environ 40 %, et de préférence entre 0,5 et environ 15 % en poids par rapport au poids de la composition ; l'agent acide peut être notamment un acide minéral tel que l'acide phosphorique, sulfamique, chlorhydrique, fluorhydrique, sulfurique, nitrique, chromique et des mélanges de ceux-ci ou un acide organique, notamment l'acide acétique, hydroxyacétique, adipique, citrique, formique, fumarique, gluconique, glutarique, glycolique, malique, maléique, lactique, malonique, oxalique, succinique et tartrique ainsi que des mélanges de ceux-ci, des sels d'acides tels que le bisulfate de sodium et des mélanges de ceux-ci ; la quantité préférée dépend du type du nettoyant acide utilisé : par exemple avec l'acide sulfamique, elle est comprise entre 0,2 et 10%, avec l'acide chlorhydrique entre 1 et 15 %, avec l'acide citrique entre 2 et 15 %, avec l'acide formique, entre 5 et 15 % et avec l'acide phosphorique, entre 2 et 30 % en poids.
- de 0,5 à 10% en poids d'au moins un agent tensioactif, de préférence anionique ou non-ionique
- éventuellement de 0,1 à 2 % en poids d'au moins un agent tensioactif cationique à propriété désinfectante (notamment mélange de n-alkyl dimethyl ethylbenzyl ammonium chloride et n-alkyl dimethyl benzyl ammonium chloride)
- éventuellement un agent épaississant (en quantité allant de 0,1 à 3%, du poids de composition), de type gomme, notamment une gomme xanthane ou un succinoglycane (Rheozan)
- éventuellement un agent de blanchiment (en quantité allant de 1 à 10%, du poids de composition)
- éventuellement un conservateur, un colorant, un parfum ou un abrasif
- et de 50 à 95 % en poids d'eau.

Ci-après sont explicités quelques autres modes particuliers de réalisation et d'application de la composition de l'invention.

Ainsi, la composition selon l'invention peut être mise en oeuvre pour le traitement nettoyant facilité de surfaces en verre, notamment de vitres. Ce traitement peut être effectué par les diverses techniques connues. On peut citer en particulier les techniques de nettoyage de vitres par pulvérisation d'un jet d'eau à l'aide d'appareils de type Karcher®.

La quantité de copolymère introduite sera généralement telle que, lors de l'utilisation de la composition de nettoyage, après dilution éventuelle, la concentration en copolymère soit comprise entre 0,001 g/l et 2 g/l, de préférence de 0,005 g/l et 0,5 g/l.

La composition de nettoyage des vitres selon l'invention comprend :

- de 0,001 à 10 %, de préférence 0,005 à 3 % en poids du copolymère;
- de 0,005 à 20 %, de préférence de 0,5 à 10 % en poids d'au moins un agent tensioactif non-ionique (par exemple un amine oxyde ou un alkyl polyglucoside) et/ou anionique ; et
- le reste étant formé d'eau et/ou d'additifs divers usuels dans le domaine.

Les formulations nettoyantes pour vitres comprenant ledit polymère peuvent également contenir :

- de 0 à 10%, avantageusement de 0,5 à 5 % de tensioactif amphotère,
- de 0 à 30 %, avantageusement de 0,5 à 15 % de solvant tels que des alcools, et
- le reste étant constitué par de l'eau et des additifs usuels (parfums notamment).
  Le pH de la composition est avantageusement compris entre 6 et 11.

La composition de l'invention est également intéressante pour le nettoyage facilité de la vaisselle en machine automatique. Ladite composition peut être soit une formule détergente (nettoyante) utilisée dans le cycle de lavage, soit une formule de rinçage. Les compositions détergentes pour lavage de la vaisselle dans des lave-vaisselle automatiques selon l'invention, comprennent avantageusement de 0,01 à 5 %, de préférence 0,1 à 3 % en poids de copolymère.

Lesdites compositions détergentes pour lave-vaisselle comprennent également au moins un agent tensioactif, de préférence non ionique en quantité pouvant aller de 0,2 à 10% de préférence de 0,5 à 5% du poids de ladite composition détergente, le reste étant constitué par des additifs divers et des charges, comme déjà mentionné ci-dessus. Ainsi elles peuvent en outre comprendre

- jusqu'à 90% en poids, d'au moins un adjuvant de détergence ("builder") de type silicate ou tripolyphosphate de sodium
- jusqu'à 10%, de préférence de 1 à 10%, tout particulièrement de 2 à 8% en poids, d'au moins un agent auxiliaire de nettoyage, un copolymère d'acide acrylique et d'acide méthyl propane sulfonique (AMPS) de préférence
- jusqu'à 30% en poids d'au moins un agent de blanchiment, de préférence perborate ou percarbonate, associé ou non à un activateur de blanchiment
- jusqu'à 50% en poids d'au moins une charge, de préférence sulfate de sodium ou chlorure de sodium

Le pH est avantageusement compris entre 8 et 13.

Les compositions pour le rinçage facilité de la vaisselle en lave-vaisselle automatique selon l'invention, peuvent comprendre avantageusement de 0,02 à 10 %, de préférence de 0,1 à 5 % en poids de copolymère par rapport au poids total de la composition.

Lesdites compositions peuvent comprendre également de 0,1 à 20 %, de préférence 0,2 à 15 % en poids par rapport au poids total de ladite composition d'un agent tensioactif, de préférence non ionique.

Parmi les agents tensioactifs non ioniques préférés, on peut citer les agents tensioactifs de type alcoylphénols en $C_6$-$C_{12}$ polyoxyéthylénés, les alcools aliphatiques en $C_8$-$C_{22}$ polyoxyéthylénés et/ou polyoxypropylénés, les copolymères bloc oxyde d'éthylène - oxyde de propylène, les amides carboxyliques éventuellement polyoxyéthylénés .... Lesdites compositions peuvent comprendre en outre de 0 à 10 %, de préférence de 0,5 à 5 % en poids par rapport au poids total de la composition d'un acide organique séquestrant du calcium, de préférence de l'acide citrique.

Elles peuvent également comprendre un agent auxiliaire de type copolymère d'acide acrylique et d'anhydride maléïque ou des homo-polymères d'acide acrylique à raison de 0 à 15 %, de préférence 0,5 à 10 % en poids par rapport au poids total de ladite composition.

Le pH est avantageusement compris entre 4 et 7.

L'invention a également pour objet une composition nettoyante pour le lavage facilité de la vaisselle à la main.

Des formulations détergentes préférées de ce type comprennent de 0,1 à 10 parties en poids de copolymère pour 100 parties en poids de ladite composition et contiennent de 3 à 50, de préférence de 10 à 40 parties en poids d'au moins un agent tensioactif, de préférence anionique, choisi notamment parmi les sulfates d'alcools aliphatiques saturés en $C_5$-$C_{24}$, de préférence en $C_8$-$C_{16}$, éventuellement condensés avec environ 0,5 à 30, de préférence 0,5 à 8, tout particulièrement 0,5 à 5 moles d'oxyde d'éthylène, sous forme acide ou sous forme d'un sel, notamment alcalin (sodium), alcalino-terreux (calcium, magnésium) ...

D'une manière préférentielle, il s'agit de formulations aqueuses détergentes liquides moussantes pour le lavage facilité à la main de la vaisselle.

Lesdites formulations peuvent en outre contenir d'autres additifs, notamment d'autres agents tensioactifs, tels que :

- des agents tensioactifs non ioniques tels que les oxydes d'amines, les alkylglucamides, les alkyl polyglucosides, les dérivés oxyalkylénés d'alcools gras, les alkylamides, les alcanolamides, des agents tensioactifs amphotères ou zwitterioniques.

- des agents bactéricides ou désinfectants non cationiques comme le triclosan
- des polymères cationiques synthétiques
- des polymères pour contrôler la viscosité du mélange et/ou la stabilité des mousses formées à l'utilisation
- des agents hydrotropes
- des agents hydratants ou humectants ou protecteurs de la peau
- des colorants, des parfums , des conservateurs, des sels divalents (notamment de magnésium) ...

Le pH de la composition est avantageusement compris entre 5 et 9.

Un autre mode de réalisation particulier de l'invention consiste en une composition de nettoyage externe facilité, notamment de la carrosserie, des véhicules motorisés (voitures, camions, autobus, trains, avions ...).

Dans ce cas également, il peut s'agir d'une composition de nettoyage proprement dit ou une composition de rinçage.

La composition nettoyante pour véhicules automobiles comprend avantageusement de 0,005 à 10 % en poids de copolymère par rapport au poids total de ladite composition, ainsi que :

- des agents tensioactifs non ioniques (à raison de 0 à 30%, de préférence de 0,1 à 15 % de la formulation),
- des agents tensioactifs amphotères et/ou zwitterioniques (à raison de 0 à 30%, de préférence de 0,01 à 10 % de la formulation)
- des agents tensioactifs cationiques (à raison de 0 à 30%, de préférence de 0,05 à 15 % de la formulation);
- des agents tensioactifs anioniques (à raison de 0 à 30%, de préférence de 0,1 à 15 % de la formulation);
- des adjuvants de détergence ("builders") (à raison de 1 à 99%, de préférence de 40 à 98 % de la formulation);
- des agents hydrotropes
- des charges, des agents régulant le pH ...

La quantité minimum d'agent tensioactif présent dans de type de composition est de préférence d'au moins 0,5% de la formulation.

Le pH de la composition est avantageusement compris entre 8 et 13.

La composition de l'invention est aussi particulièrement adaptée pour le nettoyage facilité de surfaces dures de type céramiques (carrelage, baignoires, lavabos, etc...), notamment pour salles de bain.

La formulation nettoyante comprend avantageusement de 0,02 à 5 % en poids de copolymère par rapport au poids total de ladite composition ainsi qu'au moins un agent tensioactif.

Comme agents tensioactifs, on préfère les agents tensioactifs non ioniques, notamment les composés produits par condensation de groupes oxyde d'alkylène de nature hydrophile avec un composé organique hydrophobe qui peut être de nature aliphatique ou alkyl-aromatique.

La longueur de la chaîne hydrophile ou du radical polyoxyalkylène condensée avec un groupe hydrophobe quelconque peut être facilement réglée pour obtenir un composé soluble dans l'eau ayant le degré souhaité d'équilibre hydrophile/hydrophobe (HLB). La quantité d'agents tensioactifs non ioniques dans la composition de l'invention peut être de 0 à 30 % en poids, de préférence de 0 à 20 % en poids.

Un tensioactif anionique peut éventuellement être présent en quantité de 0 à 30%, avantageusement 0 à 20% en poids.

Il est également possible mais non obligatoire d'ajouter des détergents amphotères, cationiques ou zwitterioniques.

La quantité totale de composés tensioactifs employée dans ce type de composition est généralement comprise entre 0,5 et 50 %, de préférence entre 1 et 30 % en poids, et plus particulièrement entre 2 et 20 % en poids par rapport au poids total de la composition.

Ladite composition de nettoyage peut également comprendre d'autres ingrédients minoritaires, comme :

- des adjuvants de détergence ("builders") tels que mentionnés précédemment (en quantité pouvant être comprise entre 0,1 et 25 % en poids par rapport au poids total de la composition)
- un agent de régulation de la mousse, tel que mentionné ci-dessus, notamment de type savon (en quantité généralement d'au moins 0,005 % en poids, de préférence de 0,5 % à 2 % en poids par rapport au poids total de la composition)
- des agents de régulation du pH, des colorants, des brillanteurs optiques, des agents de suspension des salissures, des enzymes détersives, des agents de blanchiment compatibles, des agents de régulation de la formation de gel, des stabilisateurs de congélation-décongélation, des bactéricides, des conservateurs, des solvants, des fongicides, des répulsifs pour insectes, des agents hydrotropes, des parfums et des opacifiants ou perlescents.

Le pH de la composition est avantageusement compris entre 2 et 12.

La composition selon l'invention convient également au rinçage facilité des parois des douches.

Les compositions aqueuses de rinçage des parois des douches comprennent de 0,02 % à 5 % en poids, avantageusement de 0,05 à 1 % du copolymère.

Les autres composants actifs principaux des compositions aqueuses de rinçage de douches de la présente invention sont au moins un agent tensioactif présent en une quantité allant de 0,5 à 5 % en poids et éventuellement un agent chélatant de métaux tel que mentionné ci-dessus, présent en une quantité allant de 0,01 à 5 % en poids.

Les compositions aqueuses de rinçage pour douches contiennent avantageusement de l'eau avec éventuellement au moins un alcool inférieur en proportion majoritaire et des additifs en proportion minoritaire (entre environ 0,1 et environ 5 % en poids, plus avantageusement entre environ 0,5 % et environ 3 % en poids, et encore plus préférentiellement entre environ 1 % et environ 2 % en poids).

Certains agents tensioactifs utilisables dans ce type d'application sont décrits dans les brevets US 5,536,452 et 5,587,022 dont le contenu est incorporé par référence dans la présente description.

Des tensioactifs préférés sont des esters gras polyéthoxylés, par exemple des mono-oléates de sorbitane polyéthoxylés et de l'huile de ricin polyéthoxylée. Des exemples particuliers de tels agents tensioactifs sont les produits de condensation de 20 moles d'oxyde d'éthylène et de mono-oléate de sorbitane (commercialisés par RHODIA Inc. sous la dénomination ALKAMULS PSMO-20® avec une HLB de 15,0) et de 30 ou 40 moles d'oxyde d'éthylène et d'huile de ricin (commercialisés par RHODIA Inc. sous la dénomination ALKAMULS EL-620 ® (HLB de 12,0) et EL-719® (HLB de 13,6) respectivement). Le degré d'éthoxylation est de préférence suffisant pour obtenir un tensioactif ayant une HLB supérieure à 13.

Le pH de la composition est avantageusement compris entre 7 et 11.

La composition selon l'invention peut également être mise en oeuvre pour le nettoyage facilité de plaques vitrocéramiques.

Avantageusement, les formulations pour le nettoyages de plaques vitrocéramiques de l'invention comprennent :

- 0,01 à 5 % en poids de copolymère;
- 0,1 à 1 % en poids d'un épaississant tel qu'une gomme xanthane ;
- 10 à 60 % en poids d'un agent abrasif tel que le carbonate de calcium ou la silice ;
- 0 à 7 % en poids d'un solvant tel que le butyldiglycol ;
- 1 à 10 % en poids d'un agent tensioactif non ionique ; et
- éventuellement des agents d'alcalinisation ou des séquestrants.

Le pH de la composition est avantageusement compris entre 7 et 12 .

Comme mentionné ci-dessus, la composition selon l'invention peut également être mise en oeuvre dans le domaine du nettoyage industriel, notamment pour le nettoyage facilité de réacteurs.

Avantageusement, lesdites compositions comprennent :

- de 0,02 à 5 % en poids de copolymère;
- de 1 à 50 % en poids de sels alcalins (phosphates, carbonates, silicates de sodium ou potassium);
- de 1 à 30 % en poids d'un mélange d'agents tensioactifs, notamment d'agents tensioactifs non-ioniques comme les alcools gras éthoxylés et les agents tensioactifs anioniques comme le lauryl benzène sulfonate ;
- de 0 à 30% en poids d'un solvant comme le diisobutyl ester.

Le pH d'une telle composition est généralement de 8 à 14 .

D'autres détails ou avantages de l'invention pourront apparaître au vu des exemples qui suivent, sans caractère limitatif.

## EXEMPLES

Exemple 1: Préparation d'un copolymère statistique de SPP et de MAPTAC 80/20 molaire (p(SPP-stat-MAPTAC) 80/20)

Dans un réacteur verré de 1 litre muni d'une agitation mécanique (ancre en téflon), d'un réfrigérant, d'une sonde de température en inox, d'une entrée d'azote et d'une double enveloppe reliée à un bain thermostaté, sont introduits en pied de cuve à température ambiante 150g de SPP (Raschig), 56,62g d'une solution aqueuse à 50% massique en MAPTAC (Röhm-Degussa) et 229,4g d'eau épurée. Le milieu réactionnel est mis sous azote (balayage) et porté à 80°C en 1 h. A 80°C une solution aqueuse de persulfate d'ammonium (0,1317g dans 10g d'eau épurée) est ajoutée dans le réacteur. La température de 80°C et l'agitation est maintenue pendant 6h.

Exemple 2: Préparation d'un copolymère statistique de SPP et de DIQUAT 90/10 molaire (p(SPP-stat-DIQUAT) 90/10)

Dans un réacteur verré de 1 litre muni d'une agitation mécanique (ancre en téflon), d'un réfrigérant, d'une sonde de température en inox, d'une entrée d'azote et d'une double enveloppe reliée à un bain thermostaté, sont introduits en pied de cuve à température ambiante 150g de SPP (Raschig), 31,4g d'une solution aqueuse à 65% massique en DIQUAT et 234,8g d'eau épurée. Le milieu réactionnel est mis sous azote (balayage) et porté à 80°C en 1 h. A 80°C une solution aqueuse de persulfate d'ammonium (0,1171g dans 10g d'eau épurée) est ajoutée dans le réacteur. La température de 80°C et l'agitation est maintenue pendant 6h.

Exemple 3: Préparation d'un copolymère statistique de SPP d'acrylamide (AM) et de DIQUAT15/60/25 molaire (p(SPP-stat-AM-DIQUAT) 15/60/25)

Dans un réacteur verré de 1 litre muni d'une agitation mécanique (ancre en téflon), d'un réfrigérant, d'une sonde de température en inox, d'une entrée d'azote et d'une double enveloppe reliée à un bain thermostaté, sont introduits en pied de cuve à température ambiante 50g de SPP (Raschig), 97,24g d'une solution aqueuse à 50% massique en AM (SNF-Floerger), 157g d'une solution aqueuse à 65% massique en DIQUAT et 187,8g d'eau épurée. Le milieu réactionnel est mis sous azote (balayage) et porté à 80°C en 1 h. A 80°C une solution aqueuse de persulfate d'ammonium (0,2341 g dans 10g d'eau épurée) est ajoutée dans le réacteur. La température de 80°C et l'agitation est maintenue pendant 6h.

Exemple 4: Préparation d'un copolymère statistique de SPE et de MAPTAC 80/20 molaire (p(SPE-stat-MAPTAC) 80/20)

Dans un réacteur verré de 1 litre muni d'une agitation mécanique (ancre en téflon), d'un réfrigérant, d'une sonde de température en inox, d'une entrée d'azote et d'une double enveloppe reliée à un bain thermostaté, sont introduits en pied de cuve à température ambiante 150g de SPE (Raschig), 59,3g d'une solution aqueuse à 50% massique en MAPTAC (Röhm-Degussa) et 230g d'eau épurée. Le milieu réactionnel est mis sous azote (balayage) et porté à 80°C en 1 h. A 80°C une solution aqueuse de persulfate d'ammonium (0,1378g dans 10g d'eau épurée) est ajoutée dans le réacteur. La température de 80°C et l'agitation est maintenue pendant 6h.

Exemple 5 (comparatif): Préparation d'un homopolymère de SPE

Dans un réacteur verré de 1 litre muni d'une agitation mécanique (ancre en téflon), d'un réfrigérant, d'une sonde de température en inox, d'une entrée d'azote et d'une double enveloppe reliée à un bain thermostaté, sont introduits en pied de cuve à température ambiante 150g de SPE (Raschig) et 215,2g d'eau épurée. Le milieu réactionnel est mis sous azote (balayage) et porté à 80°C en 1 h. A 80°C une solution aqueuse de persulfate d'ammonium (0,1103g dans 10g d'eau épurée) est ajoutée dans le réacteur. La température de 80°C et l'agitation est maintenue pendant 6h.

Exemples 6-15 - Préparation de shampooings comprenant les (co)polymères

On réalise des compositions de shampooings comprenant des ingrédients choisis parmi les suivants:

| Matière première | Source | Nature |
|---|---|---|
| SLES | laurylethersulfate de sodium (2EO), EMPICOL ESB/3M commercialisé par Hunstman | Tensioactif anionique |
| CAPB | Cocoamidopropylbétaïne, MIRATAINE BET-C-30 commercialisé par Rhodia | Tensioactif amphotère |
| NaCl | | Sel |
| Polymère | Polymère selon les exemples 1 à 5 | |
| Silicone 1 | Mirasil DME-2 commercialisée par Rhodia: émulsion de dimethicone (PDMS) de viscosité d'environ 500000 cP, de taille des gouttelettes d'environ 2 $\mu$m, stabilisée par du succinoglycane | |

Mode opératoire

1. Mélanger l'eau et le polymère
2. Ajouter le CAPB
3. Ajouter le tensioactif anionique, puis éventuellement l'émulsion de silicone
4. Ajuster le pH à 6-6,5 par ajout de d'hydroxyde de sodium ou d'acide citrique
5. Ajouter le sel

On réalise les compositions suivantes, dont la quantité en poids de chaque ingrédient (matière sèche) est donnée ci-dessous (la lettre C indique des exemples comparatifs):

| Exemple | 6 | 7 | 8 | 9 | 10C |
|---|---|---|---|---|---|
| SLES (%) | 8 | 8 | 8 | 8 | 8 |
| CAPB (%) | 4 | 4 | 4 | 4 | 4 |
| NaCl (%) | 0,2 | 0,2 | 0,2 | 0,2 | 0,2 |
| Polymère | Exemple 1 0,2% | Exemple 2 0,2% | Exemple 3 0,2% | Exemple 4 0,2% | Exemple 5 0,2% |
| Eau | Jusqu'à 100% | | | | |

| Exemple | 11 | 12 | 13 | 14 | 15C |
|---|---|---|---|---|---|
| SLES (%) | 8 | 8 | 8 | 8 | 8 |
| CAPB (%) | 4 | 4 | 4 | 4 | 4 |
| NaCl (%) | 0,2 | 0,2 | 0,2 | 0,2 | 0,2 |
| Silicone 1 (% de matière sèche) | 1 | 1 | 1 | 1 | 1 |
| Polymère | Exemple 1 0,2% | Exemple 2 0,2% | Exemple 3 0,2% | Exemple 4 0,2% | Exemple 5 0,2% |
| Eau | Jusqu'à 100% | | | | |

<u>Exemples 16-17</u>: Tests de dilution

On étudie le comportement des compositions lors d'une dilution avec de l'eau, par suivi de la transmittance.

La transmittance est mesurée à l'aide de l'appareil Photometer 662 de Metrohm à une longueur d'onde de 600 nm, sur des cellules en quartz de 1 cm de large.

La procédure de mesure lors d'une dilution est la suivante:

Une dilution de facteur 2 correspond à une dilution d'une part en poids de composition testée avec une part en poids d'eau (1 part + 1 part = dilution 2). La composition est placée dans un bécher muni d'une barre d'agitation magnétique. L'au y est ajoutée. La sonde de l'appareil de mesure de la transmittance est placée dans le mélange. La barre d'agitation est mise en mouvement de manière à agiter sans créer de bulle (qui pourraient altérer la mesure de transmittance). Après 5 minutes d'agitation le % de transmittance est noté. Cette opération est répétée four un facteur de dilution 4 (1 part de composition pour 3 parts d'eau), 6, 8 etc. La transmittance à divers taux de dilution (facteur de dilution) est reportée sur le tableau ci-dessous.

| | Exemple 16 | Exemple 17 |
|---|---|---|
| Facteur de dilution | Transmittance, Composition de l'exemple 8 | Transmittance, Composition de l'exemple 6 |
| 0 | 96,3 | 98,8 |
| 2 | 1 | 96,5 |
| 4 | 1,8 | 21,8 |
| 6 | 5,1 | 22,6 |

(suite)

| | Exemple 16 | Exemple 17 |
|---|---|---|
| Facteur de dilution | Transmittance, Composition de l'exemple 8 | Transmittance, Composition de l'exemple 6 |
| 8 | 10,7 | 99,4 |

L'abaissement de la transmittance correspond à la formation de coacervats. A forte dilution, les coacervats sont complètement déstabilisés et tombent au fond de la cellule. Ces coacervats procurent un conditionnement approprié sur les cheveux ou une aide au conditionnement appropriée.

## Revendications

**1.** Copolymère comprenant des unités zwitterioniques A et d'autres unités B, les unités A comprenant un groupe bétaïne, **caractérisé en ce que**:

- les unités B sont des unités cationiques ou des unités potentiellement cationiques comprenant au moins un groupe amine ternaire, et
- les unités A répondent à l'une des formules suivantes:

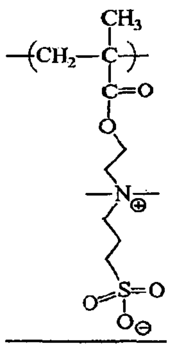

-(SPE)-

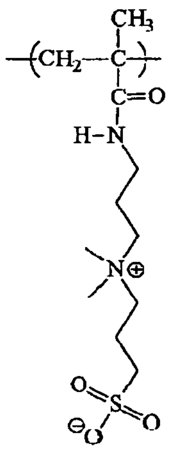

-(SPP)-

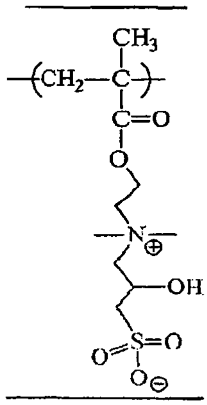

-(SHPE)-

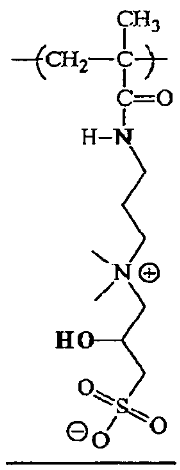

-(SHPP)-

**2.** Copolymère selon la revendication 1, **caractérisé en ce que** le rapport molaire entre les unités A et les unités B est compris entre 99/1 et 1/99, de préférence entre 90/10 et 50/50.

**3.** Copolymère selon l'une des revendications précédentes **caractérisé en ce qu'**il comprend eh plus:

- des unités $C_N$ non ioniques, hydrophiles ou hydrophobes, et/ou
- des unités $C_A$ anioniques ou potentiellement anioniques.

**4.** Copolymère selon l'une des revendications précédentes, **caractérisé en ce que** les unités A et B représentent de 1% à 100%, de préférence de 1 à 95% en moles des unités du copolymère.

**5.** Copolymère selon l'une des revendications précédentes, **caractérisé en ce qu'**il s'agit:

- d'un copolymère statistique,
- d'un copolymère à gradient,
- d'un copolymère peigne,
- d'un copolymère à blocs comprenant un bloc comprenant les unités A et un bloc comprenant les unités B,
- d'un copolymère à blocs comprenant un bloc comprenant les unités A et B, et un bloc différent ne comprenant pas à la fois des unités A et des unités B, de préférence un bloc différent comprenant:

    - des unités $C_N$ non ioniques, hydrophiles ou hydrophobes, et/ou
    - des unités $C_A$ anioniques ou potentiellement anioniques.

**6.** Copolymère selon l'une des revendications précédentes, **caractérisé en ce que** les groupes bétaïnes des unités de A sont groupes pendants du copolymère.

**7.** Copolymère selon l'une des revendications précédentes, **caractérisés en ce que** les charges présentes dans le copolymère sont portées par des groupes pendants.

**8.** Copolymère selon l'une des revendications précédentes, **caractérisé en ce que** les unités A, B, avec éventuellement d'autres unités, forment une chaîne hydrocarbonée polyalkylène éventuellement interrompue par un ou plusieurs atomes d'azote ou de soufre.

**9.** Copolymère selon l'une des revendications précédentes, **caractérisé en ce que** les unités B sont des unités cationiques ou potentiellement cationiques comprenant au 1, 2, 3, ou plus, groupes cationiques ou potentiellement cationiques, dans la chaîne formant le squelette du copolymère ou en position latérale par rapport à la chaîne formant le squelette du copolymère.

**10.** Copolymère selon l'une des revendications précédentes, **caractérisé en ce que** les unités B cationiques sont des unités comprenant au moins un groupe ammonium quaternaire.

**11.** Copolymère selon l'une des revendications précédentes, **caractérisé en ce que** les unités B sont choisies parmi les unités dérivant de monomères B cationiques ou potentiellement cationiques suivants:

- N,N-diméthylaminométhyl-acrylamide ou -méthacrylamide,
- le 2(N,N-diméthylamino)éthyl-acrylamide ou -méthacrylamide,
- le 3(N,N-diméthylamino)propyl-acrylamide ou -méthacrylamide,
le 4(N,N-diméthylamino)butyl-acrylamide ou -méthacrylamide
le 2(diméthyl amino)éthyl acrylate (ADAM),
- le 2(dimethyl amino)éthyl méthacrylate (DMAM),
- le 3(diméthyl amino)propyl méthacrylate, le 2(tertiobutylamino)éthyl méthacrylate,
- le 2(dipentylamino)éthyl méthacrylate,
- le 2(diéthylamino)éthyl méthacrylate
- les vinylpyridines
- la vinyl amine
- les vinylimidazolines
- le chlorure de triméthylammoniumpropylméthacrylate,
- le chlorure ou le bromure de triméthylammoniuméthylacrylamide ou méthacrylamide,
- le méthylsulfate de triméthylammoniumbutylacrylamide ou méthacrylamide,
- le méthylsulfate de triméthylammoniumpropylméthacrylamide (MES),
- le chlorure de (3-méthacrylamidopropyl)triméthylammonium (MAPTAC),
- le chlorure de (3-acrylamidopropyl)triméthylammonium (APTAC),
- le chlorure ou le méthylsulfate de méthacryloyloxyéthyl triméthylammonium,
- le chlorure d'acryloyloxyéthyl triméthylammonium ou le méthylsulfate d'acryloyloxyéthyl triméthylammonium (ADAMQUAT Cl ou ADAMQUAT MeS),
- le méthyle sulfate de méthyldiethylammonium éthyle acrylate (ADAEQUAT MeS),
- le chlorure ou méthyle sulfate de benzyldimethylammonium éthyle acrylate (ADAMQUAT BZ 80).
- le bromure, chlorure ou méthylsulfate de 1-éthyl 2-vinylpyridinium, de 1-éthyl 4-vinylpyridinium ;
- le chlorure de N,N-diméthyldiallylammonium (DADMAC) ;
- le chlorure de diméthylaminopropylméthacrylamide,N-(3-chloro-2-hydroxypropyl) triméthylammonium (DIQUAT chlorure),
- le methylsulfate de diméthylaminopropylméthacrylamide,N-(3-methylsulfate-2-hydroxypropyl) triméthylammonium (DIQUAT methylsulfate)
- le monomère de formule

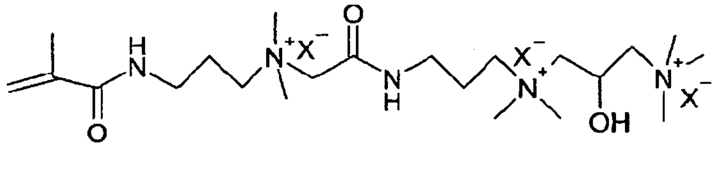

où $X^-$ est un anion, de préférence chlorure ou méthylsulfate.

**12.** Copolymère selon l'une des revendications précédentes, **caractérisé en ce qu'**il comprend des unités $C_N$ non ioniques hydrophiles choisies parmi les unités dérivant de monomères $C_N$ non ioniques hydrophiles suivants:

- les hydroxyalkylesters d'acides α-β éthyléniquement insaturés comme les acrylates et méthacrylates d'hydroxyéthyle, d'hydroxypropyle, le glycérol monométhacrylate...
- les amides α-β éthyléniquement insaturés comme l'acrylamide, le méthacrylamide, le N-méthylolacrylamide,
- les monomères α-β éthyléniquement insaturés portant un segment polyoxyalkyléné hydrosoluble du type polyoxyde d'éthylène, comme les polyoxyde le cas échéant statistique ou à blocs d'éthylène et/ou de propylène α-méthacrylates ou α,ω-diméthacrylates, le méthacrylate de polyoxyéthylène ω-béhényle éventuellement en mélange, le méthacrylate de polyoxyéthylène ω-tristyrylphényle,
- les monomères α-β éthyléniquement insaturés précurseurs d'unités ou de segments hydrophiles tels que l'acétate de vinyle qui, une fois polymérisés, peuvent être hydrolysés pour engendrer des unités alcool vinylique ou des segments alcool polyvinylique,
- les vinylpyrrolidones,
- les monomères α-β éthyléniquement insaturés de type uréido et en particulier le méthacrylamido de 2-imidazolidinone éthyle éventuellement en mélange,
- le nonethyleneglycolmethyletheracrylate ou le nonethyleneglycolmethylethermethacrylate.

**13.** Copolymère selon l'une des revendications précédentes, **caractérisé en ce qu'**il s'agit:

- d'un copolymère dérivant de
  - A: SPE, de préference de 5 à 95 mol%, plus préférablement de 10 à 90%, et
  - B: MAPTAC, de préference de 5 à 95 mol%, plus préférablement de 10 à 90%,
- d'un copolymère dérivant de
  - A: SPE, de préference de 5 à 95 mol%, plus préférablement de 10 à 90 mol%, et
  - B: DIQUAT, de préference de 5 à 95 mol%, plus préférablement de 10 à 90 mol%,
- d'un copolymère dérivant de
  - A: SPE, de préférence de 5 à 90 mol%, plus préférablement de 10 à 80 mol%,
  - B: MAPTAC, de préférence de 5 à 90 mol%, plus préférablement de 10 à 80 mol%, et
  - C: acrylamide, de préférence de 5 à 90 mol%, plus préférablement de 10 à 80 mol%,
- d'un copolymère dérivant de
  - A: SPE. de préférence de 5 à 90 mol%, plus préférablement de 10 à 80 mol%,
  - B: DIQUAT, de préférence de 5 à 90 mol%, plus préférablement de 10 à 80 mol%, et
  - C: acrylamide, de préférence de 5 à 90 mol%, plus préférablement de 10 à 80 mol%,
- d'un copolymère dérivant de
  - A: SPP, de préférence de 5 à 95 mol%, plus préférablement de 10 à 90 mol%, et
  - B: MAPTAC, de préférence 5 à 95 mol%, plus préférablement de 10 à 90 mol%,
- d'un copolymère dérivant de
  - A: SPP, de préférence de 5 à 95 mol%, plus préférablement de 10 à 90 mol%, et
  - B: DIQUAT, de préférence de 5 à 95 mol%, plus préférablement de 10 à 90 mol%,
- d'un copolymère dérivant de
  - A: SPP, de préférence de 5 à 90 mol%, plus préférablement de 10 à 80 mol%,
  - B: MAPTAC, de préférence de 5 à 90 mol%, plus préférablement de 10 à 80 mol%, et
  - C: acrylamide, de préférence de 5 à 90 mol%, plus préférablement de 10 à 80 mol%, ou

- d'un copolymère dérivant de

  - A: SPP, de préférence de 5 à 90 mol%, plus préférablement de 10 à 80 mol%,
  - B: DIQUAT, de préférence de 5 à 90 mol%, plus préférablement de 10 à 80 mol%, et
  - C: acrylamide, de préférence de 5 à 90 mol%, plus préférablement de 10 à 80 mol%.

**14.** Copolymère selon l'une des revendications précédentes, **caractérisé en ce qu'**il est hydrosoluble ou hydrodispersable.

**15.** Composition pour le traitement ou la modification de surfaces comprenant:

- un vecteur, de préférence liquide,
- le copolymère selon l'une des revendications précédentes,
- éventuellement un tensioactif,
- éventuellement un sel, un acide et/ou une base, et
- éventuellement un agent pour le traitement ou la modification de la surface.

**16.** Composition selon la revendication 15, **caractérisée en ce qu'**elle comprend un tensioactif anionique ou amphotère.

**17.** Composition selon la revendication 15, **caractérisée en ce qu'**elle comprend un tensioactif non ionique.

**18.** Composition selon l'une des revendications 15 à 17, **caractérisée en ce que** les unités B sont sous forme cationiques au pH de la composition.

**19.** Composition selon l'une des revendications 15 à 19, **caractérisée en ce que**:

- elle comprend un tensioactif anionique ou amphotère, et
- elle comprend des coacervats ou elle forme des coacervats par dilution et/ou modification du pH.

**20.** Composition selon la revendication 19, **caractérisée en ce que** le copolymère, le tensioactif anionique ou amphotère, le vecteur, éventuellement d'autres tensioactifs, un sel, un base et/ou un acide, et leurs quantités, sont tels que au moins une partie de système constitué du copolymère, du tensioactif anionique ou amphotère, du vecteur, et éventuellement des autres tensioactifs, sel, base et/ou acide,

- forme des coacervats par dilution et/ou modification du pH, ou
- comprend des coacervats.

**21.** Composition selon l'une des revendications 15 à 20, **caractérisée en ce que** elle comprend un agent pour le traitement ou la modification de la surface, ledit agent étant un polyorganosiloxane, un actif anti-pelliculaire, un parfum, une huile, ou un filtre UV.

**22.** Composition selon l'une des revendications 15 à 21, **caractérisée en ce qu'**il s'agit:

- d'une composition cosmétique, de préférence destinée à être rincée, de préférence un shampooing, un produit de mise en forme des cheveux, un après-shampooing, un produit de soin des cheveux, un produit de soin de la peau ou un gel douche, ou
- d'une composition détergente pour les soins ménagers, industriels ou institutionnels, de préférence pour le soin du linge ou pour le nettoyage ou le traitement de surfaces dures.

**23.** Procédé de traitement ou de modification d'une surface, comprenant les étapes suivantes:

- appliquer sur la surface une composition selon l'une des revendications 15 à 22, et
- éventuellement, éliminer le vecteur ou diluer la composition ou modifier le pH.

**24.** Procédé selon la revendication 23, **caractérisé en ce que** la surface est la peau et/ou les cheveux, le traitement ou la modification étant un conditionnement de la peau et/ou des cheveux.

## Claims

1. Copolymer comprising zwitterionic units A and other units B, the units A comprising a betaine group, **characterized in that**:

   - the units B are cationic or potentially cationic units comprising at least one tortiary amine group, and
   - the units A correspond to one of the following formulae:

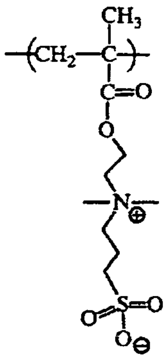

-(SPE)-

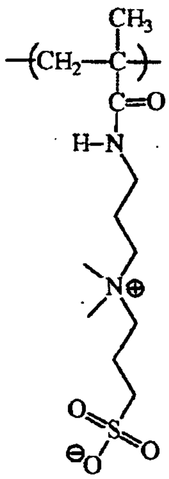

-(SPP)-

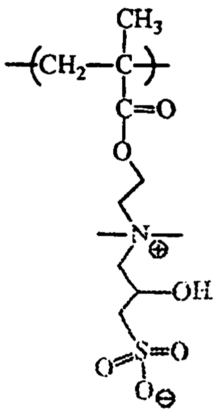

-(SHPE)-

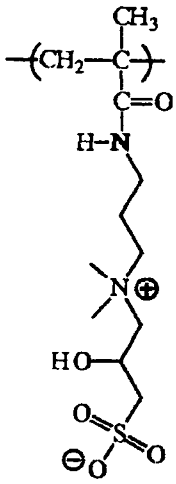

-(SHPP)-

2. Copolymer according to Claim 1, **characterized in that** the molar ratio of the units A to the units B is between 99/1 and 1/99, preferably between 90/10 and 50/50.

3. Copolymer according to either of the preceding claims, **characterized in that** it additionally comprises:

- hydrophilic or hydrophobic nonionic units $C_N$,
and/or
- anionic or potentially anionic units $C_A$.

4. Copolymer according to one of the preceding claims, **characterized in that** the units A and B represent from 1 to 100 mol%, preferably from 1 to 95 mol%, of the units of the copolymer.

5. Copolymer according to one of the preceding claims, **characterized in that** it is:

- a random copolymer,
- a gradient copolymer,
- a comb copolymer,
- a block copolymer comprising a block comprising units A and a block comprising units B,
- a block copolymer comprising a block comprising units A and B and a different block not simultaneously comprising units A and units B, preferably a different block comprising:
- hydrophilic or hydrophobic nonionic units $C_N$,
and/or
- anionic or potentially anionic units $C_A$.

6. Copolymer according to one of the preceding claims, **characterized in that** the betaine groups of the units A are pendent groups of the copolymer.

7. Copolymer according to one of the preceding claims, **characterized in that** the charges present in the copolymer are carried by pendent groups.

8. Copolymer according to one of the preceding claims, **characterized in that** the units A and B, optionally with other units, form a polyalkylene hydrocarbon chain optionally interrupted by one or more nitrogen or sulfur atoms.

9. Copolymer according to one of the preceding claims, **characterized in that** the units B are cationic or potentially cationic units comprising 1, 2, 3 or more cationic or potentially cationic groups in the chain forming the backbone of the copolymer or in the side position with respect to the chain forming the backbone of the copolymer.

10. Copolymer according to one of the preceding claims, **characterized in that** the cationic units B are units comprising at least one quaternary ammonium group.

**11.** Copolymer according to one of the preceding claims, **characterized in that** the units B are chosen from units deriving from the following cationic or potentially cationic monomers B:

- N,N-dimethylaminomethylacrylamide or -methacrylamide,
- [2-(N,N-dimethylamino)ethyl]acrylamide or -methacrylamide,
- [3-(N,N-dimethylamino)propyl]acrylamide or -methacrylamide,
- [4-(N,N-dimethylamino)butyl]acrylamide or -methacrylamide,
- 2-(dimethylamino)ethyl acrylate (ADAM),
- 2-(dimethylamino)ethyl methacrylate (DMAM),
- 3-(dimethylamino)propyl methacrylate,
- 2-(tert-butylamino)ethyl methacrylate,
- 2-(dipentylamino)ethyl methacrylate,
- 2-(diethylamino)ethyl methacrylate,
- vinylpyridines,
- vinylamine,
- vinylimidazolines,
- trimethylammoniopropyl methacrylate chloride,
- trimethylammonioethylacrylamide or -methacrylamide chloride or bromide,
- trimethylammoniobutylacrylamide or -methacrylamide methyl sulfate,
- trimethylammoniopropylmethacrylamide methyl sulfate (MES),
- (3-methacrylamidopropyl)trimethylammonium chloride (MAPTAC),
- (3-acrylamidopropyl)trimethylammonium chloride (APTAC),
- methacryloyloxyethyltrimethylammonium chloride or methyl sulfate,
- acryloyloxyethyltrimethylammonium chloride or acryloyloxyethyltrimethylammonium methyl sulfate (ADAMQUAT Cl or ADAMQUAT MeS),
- methyldiethylammonioethyl acrylate methyl sulfate (ADAEQUAT MeS),
- benzyldimethylammonioethyl acrylate chloride or methyl sulfate (ADAMQUAT BZ 80),
- 1-ethyl-2-vinylpyridinium or 1-ethyl-4-vinylpyridinium bromide, chloride or methyl sulfate,
- N,N-dimethyldiallylammonium chloride (DADMAC),
- the chloride of dimethylaminopropyl-methacrylamide, N-(3-chloro2-hydroxypropyl)-trimethylammonium (DIQUAT chloride),
- the methyl sulfate of dimethylamino-propylmethacrylamide, N-(3-(methyl sulfate)-2-hydroxypropyl)trimethylammonium (DIQUAT methyl sulfate),
- the monomer of formula:

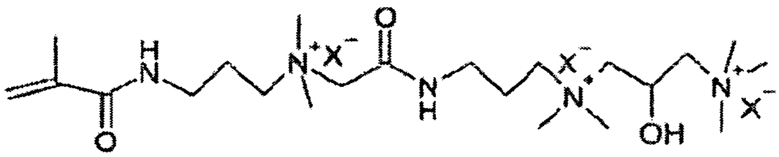

where $X^-$ is an anion, preferably chloride or methyl sulfate.

**12.** Copolymer according to one of the preceding claims, **characterized in that** it comprises hydrophilic nonionic units $C_N$ chosen from units deriving from the following hydrophilic nonionic monomers $C_N$:

- hydroxyalkyl esters of α,β-ethylenically unsaturated acids, such as hydroxyethyl acrylate, hydroxyethyl methacrylate, hydroxypropyl acrylate, hydroxypropyl methacrylate, glycerol monomethacrylate, and the like,
- α,β-ethylenically unsaturated amides, such as acrylamide, methacrylamide or N-methylolacrylamide,
- α,β-ethylenically unsaturated monomers carrying a water-soluble polyoxyalkylene segment of the polyethylene oxide type, such as, if appropriate random or block, polyethylene oxide and/or propylene oxide α-methacrylates or α,ω-dimethacrylates, ω-behenyl polyoxyethylene methacrylate, optionally as a mixture, ω-tristyrylphenyl polyoxyethylene methacrylate,
- α,β-ethylenically unsaturated monomers which are precursors of hydrophilic units or segments, such as vinyl acetate, which, once polymerized, can be hydrolyzed to produce vinyl alcohol units or polyvinyl alcohol segments,
- vinylpyrrolidones,
- α,β-ethylenically unsaturated monomers of ureido type and in particular the methacrylamido of 2-imidazolidinone ethyl, optionally as a mixture,
• nonethylene glycol methyl ether acrylate or nonethylene glycol methyl ether methacrylate.

**13.** Copolymer according to one of the preceding claims, **characterized in that** it is:

- a copolymer deriving from:

  - A: SPE, preferably from 5 to 95 mol%, more preferably from 10 to 90 mol%, and
  - B: MAPTAC, preferably from 5 to 95 mol%, more preferably from 10 to 90 mol%,

- a copolymer deriving from:

  - A: SPE, preferably from 5 to 95 mol%, more preferably from 10 to 90 mol%, and
  - B: DIQUAT, preferably from 5 to 95 mol%, more preferably from 10 to 90 mol%,

- a copolymer deriving from:

  - A: SPE, preferably from 5 to 90 mol%, more preferably from 10 to 80 mol%,
  - B: MAPTAC, preferably from 5 to 90 mol%, more preferably from 10 to 80 mol%, and
  - C: acrylamide, preferably from 5 to 90 mol%, more preferably from 10 to 80 mol%,

- a copolymer deriving from:

  - A: SPE, preferably from 5 to 90 mol%, more preferably from 10 to 80 mol%,
  - B: DIQUAT, preferably from 5 to 90 mol%, more preferably from 10 to 80 mol%, and
  - C: acrylamide, preferably from 5 to 90 mol%, more preferably from 10 to 80 mol%,

- a copolymer deriving from:

  - A: SPP, preferably from 5 to 95 mol%, more preferably from 10 to 90 mol%, and
  - B: MAPTAC, preferably from 5 to 95 mol%, more preferably from 10 to 90 mol%, and

- a copolymer deriving from:

  - A: SPP, preferably from 5 to 95 mol%, more preferably from 10 to 90 mol%,
  - B: DIQUAT, preferably from 5 to 95 mol%, more preferably from 10 to 90 mol%,

- a copolymer deriving from:

  - A: SPP, preferably from 5 to 90 mol%, more preferably from 10 to 80 mol%,
  - B: MAPTAC, preferably from 5 to 90 mol%, more preferably from 10 to 80 mol%, and
  - C: acrylamide, preferably from 5 to 90 mol%, more preferably from 10 to 80 mol%, or

- a copolymer deriving from:

  - A: SPP, preferably from 5 to 90 mol%, more preferably from 10 to 80 mol%,
  - B: DIQUAT, preferably from 5 to 90 mol%, more preferably from 10 to 80 mol%, and
  - C: acrylamide, preferably from 5 to 90 mol%, more preferably from 10 to 80 mol%.

**14.** Copolymer according to one of the preceding claims, **characterized in that** it is water-soluble or water-dispersible.

**15.** Composition for the treatment or modification of surfaces, comprising:

- a carrier, preferably a liquid carrier,
- the copolymer according to one of the preceding claims,
- optionally a surfactant,
- optionally a salt, an acid and/or a base, and
- optionally an agent for the treatment or modification of the surface.

**16.** Composition according to Claim 15, **characterized in that** it comprises an anionic or amphoteric surfactant.

**17.** Composition according to Claim 15, **characterized in that** it comprises a nonionic surfactant.

**18.** Composition according to one of Claims 15 to 17, **characterized in that** the units B are in the cationic form at the pH of the composition.

**19.** Composition according to one of Claims 15 to 19, **characterized in that**:

- it comprises an anionic or amphoteric surfactant, and
- it comprises coacervates or forms coacervates by dilution and/or modification of the pH.

**20.** Composition according to Claim 19, **characterized in that** the copolymer, the anionic or amphoteric surfactant, the carrier, optionally other surfactants, a salt, a base and/or acid, and their amounts, are such that at least a portion of system composed of the copolymer, of the anionic or amphoteric surfactant, of the carrier, and optionally the other surfactants, salt, base and/or acid,

- forms coacervates by dilution and/or modification of the pH, or
- comprises coacervates.

**21.** Composition according to one of Claims 15 to 20, **characterized in that** it comprises an agent for the treatment or modification of the surface, said agent being a polyorganosiloxane, an antidandruff active principle, a fragrance, an oil or a UV screening agent.

**22.** Composition according to one of Claims 15 to 21, **characterized in that** it is:

- a cosmetic composition, preferably intended to be rinsed out, preferably a shampoo, a hair shaping product, a conditioner, a hair care product, a skincare product or a shower gel, or
- a detergent composition for domestic, industrial or institutional care purposes, preferably for caring for the laundry or for cleaning or treating hard surfaces.

**23.** Process for the treatment or modification of a surface, comprising the following stages:

- applying, to the surface, a composition according to one of Claims 15 to 22, and
- optionally removing the carrier or diluting the composition or modifying the pH.

**24.** Process according to Claim 23, **characterized in that** the surface is the skin and/or hair, the treatment or modification being a conditioning of the skin and/or hair.

## Patentansprüche

**1.** Copolymer, umfassend zwitterionische Einheiten A und andere Einheiten B, wobei die Einheiten A eine Betaingruppe umfassen, **dadurch gekennzeichnet, dass**:

- es sich bei den Einheiten B um kationische Einheiten oder potenziell kationische Einheiten mit mindestens einer tertiären Amingruppe handelt und
- die Einheiten A einer der folgenden Formeln entsprechen:

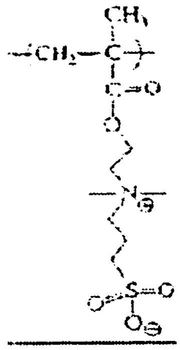

-(SPE)-

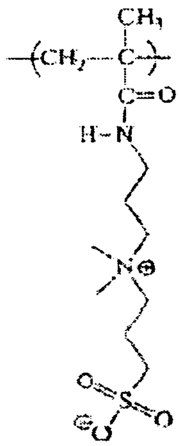

-(SPP)-

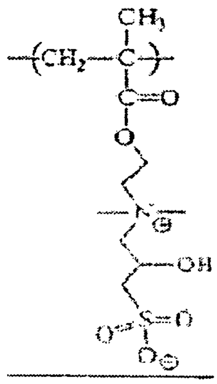

-(SHPE)-

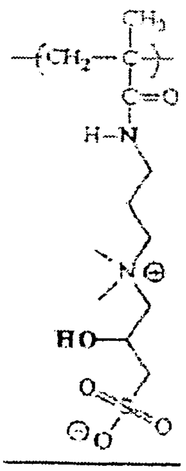

-(SHPP)-

2. Copolymer nach Anspruch 1, **dadurch gekennzeichnet, dass** das Molverhältnis zwischen den Einheiten A und den Einheiten B zwischen 99/1 und 1/99 und vorzugsweise zwischen 90/10 und 50/50 liegt.

3. Copolymer nach einem der vorhergehenden Ansprüche, **dadurch gekennzeichnet, dass** es außerdem:

   - hydrophile oder hydrophobe nichtionische Einheiten $C_N$ und/oder
   - anionische oder potenziell anionische Einheiten $C_A$

   umfasst.

4. Copolymer nach einem der vorhergehenden Ansprüche, **dadurch gekennzeichnet, dass** die Einheiten A und B 1 bis 100 Mol-% und vorzugsweise 1 bis 95 Mol-% der Einheiten des Copolymers ausmachen.

5. Copolymer nach einem der vorhergehenden Ansprüche, **dadurch gekennzeichnet, dass** es sich um:

   - ein statistisch aufgebautes Copolymer,
   - ein Gradientencopolymer,
   - ein Kammcopolymer,
   - ein Blockcopolymer mit einem Block, der Einheiten A umfasst, und einem Block, der Einheiten B umfasst,
   - ein Blockcopolymer mit einem Block, der Einheiten A und B umfasst, und einem anderen Block, der nicht gleichzeitig Einheiten A und Einheiten B umfasst, vorzugsweise einem anderen Block, der:

     - hydrophile oder hydrophobe nichtionische Einheiten $C_N$ und/oder
     - anionische oder potenziell anionische Einheiten $C_A$

     umfasst,

   handelt.

6. Copolymer nach einem der vorhergehenden Ansprüche, **dadurch gekennzeichnet, dass** es sich bei den Betaingruppen der Einheiten A um seitenständige Gruppen des Copolymers handelt.

7. Copolymer nach einem der vorhergehenden Ansprüche, **dadurch gekennzeichnet, dass** die in dem Copolymer vorliegenden Ladungen von seitenständigen Gruppen getragen werden.

8. Copolymer nach einem der vorhergehenden Ansprüche, **dadurch gekennzeichnet, dass** die Einheiten A und B gegebenenfalls mit anderen Einheiten eine Polyalkylenkohlenwasserstoffkette, die gegebenenfalls durch ein oder mehrere Stickstoff- oder Schwefelatome unterbrochen ist, bilden.

9. Copolymer nach einem der vorhergehenden Ansprüche, **dadurch gekennzeichnet, dass** es sich bei den Einheiten B um kationische oder potenziell kationische Einheiten mit 1, 2, 3 oder mehr kationischen oder potenziell kationischen Gruppen in der das Gerüst des Copolymers bildenden Kette oder in lateraler Position bezüglich der das Gerüst des Copolymers bildenden Kette handelt.

**10.** Copolymer nach einem der vorhergehenden Ansprüche, **dadurch gekennzeichnet, dass** es sich bei den kationischen Einheiten B um Einheiten mit mindestens einer quaternären Ammoniumgruppe handelt.

**11.** Copolymer nach einem der vorhergehenden Ansprüche, **dadurch gekennzeichnet, dass** die Einheiten B unter Einheiten ausgewählt sind, die sich von den folgenden kationischen oder potenziell kationischen Monomeren B abgleiten:

- N,N-Dimethylaminomethylacrylamid oder -methacrylamid,
- [2-(N,N-Dimethylamino)ethyl]acrylamid oder -methacrylamid,
- [3-(N,N-Dimethylamino)propyl]acrylamid oder -methacrylamid,
- [4-(N,N-Dimethylamino)butyl]acrylamid oder -methacrylamid,
- 2-(Dimethylamino)ethylacrylat (ADAM),
- 2-(Dimethylamino)ethylmethacrylat (DMAM),
- 3-(Dimethylamino)propylmethacrylat,
- 2-(tert.-Butylamino)ethylmethacrylat,
- 2-(Dipentylamino)ethylmethacrylat,
- 2-(Diethylamino)ethylmethacrylat,
- Vinylpyridinen,
- Vinylamin,
- Vinylimidazolinen,
- Trimethylammoniopropylmethacrylatchlorid,
- Trimethylammonioethylacrylamid oder -methacrylamidchlorid oder -bromid,
- Trimethylammoniobutylacrylamid oder -methacrylamidmethylsulfat,
- Trimethylammoniopropylmethacrylamidmethylsulfat (MES),
- (3-Methacrylamidopropyl)trimethylammoniumchlorid (MAPTAC),
- (3-Acrylamidopropyl)trimethylammoniumchlorid (APTAC),
- Methacryloyloxyethyltrimethylammoniumchlorid oder -methylsulfat,
- Acryloyloxyethyltrimethylammoniumchlorid oder Acryloyloxyethyltrimethylammoniummethylsulfat (ADAMQUAT Cl oder ADAMQUAT MeS),
- Methyldiethylammonioethylacrylatmethylsulfat (ADAEQUAT MeS),
- Benzyldimethylammonioethylacrylatchlorid oder -methylsulfat (ADAMQUAT BZ 80),
- 1-Ethyl-2-vinylpyridinium- oder 1-Ethyl-4-vinylpyridiniumbromid, -chlorid oder -methylsulfat,
- N,N-Dimethyldiallylammoniumchlorid (DADMAC),
- Dimethylaminopropylmethacrylamid-N-(3-chlor-2-hydroxypropyl)trimethylammoniumchlorid (DIQUAT-Chlorid),
- Dimethylaminopropylmethacrylamid-N-(3-methylsulfat-2-hydroxypropyl)trimethylammoniummethylsulfat (DIQUAT-Methylsulfat),
- dem Monomer der Formel

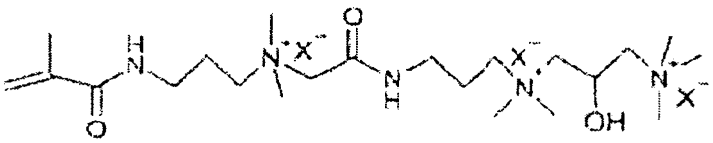

wobei $X^-$ für ein Anion, vorzugsweise Chlorid oder Methylsulfat, steht.

**12.** Copolymer nach einem der vorhergehenden Ansprüche, **dadurch gekennzeichnet, dass** es hydrophile nichtionische Einheiten $C_N$ umfasst, die unter Einheiten ausgewählt sind, die sich von den folgenden hydrophilen nichtionischen Monomeren $C_N$ ableiten:

• Hydroxyalkylestern von α,β-ethylenisch ungesättigten Säuren, wie Hydroxyethylacrylat, Hydroxyethylmethacrylat, Hydroxypropylacrylat, Hydroxypropylmethacrylat, Glycerinmonomethacrylat usw.,
• α,β-ethylenisch ungesättigten Amiden, wie Acrylamid, Methacrylamid oder N-Methylolacrylamid,
• α,β-ethyleniisch ungesättigten Monomeren mit einem wasserlöslichen Polyoxyalkylen-Segment vom Polyethylenoxid-Typ, wie gegebenenfalls statistisch oder blockartig aufgebaute Polyethylenoxid- und/oder Polypropylenoxid-α-methacrylate oder -α,ω-dimethacrylate, ω-Behenylpolyoxyethylenmethacrylat, gegebenenfalls im Gemisch, ω-Tristyrylphenylpolyoxyethylenmethacrylat,

- α, β-ethylenisch ungesättigten Monomeren, bei denen es sich um Vorläufer von hydrophilen Einheiten oder Segmenten handelt, wie Vinylacetat, das nach Polymerisation zu Vinylalkoholeinheiten oder Polyvinylalkoholsegmenten hydrolysiert werden kann,
- Vinylpyrrolidonen,
- α, β-ethylenisch ungesättigten Monomeren vom Ureido-Typ und insbesondere 2-Imidazolidinonethylmethacrylamid, gegebenenfalls im Gemisch,
- Nichtethylenglykolmethyletheracrylat oder Nichtethylenglykolmethylethermethacrylat.

**13.** Copolymer nach einem der vorhergehenden Ansprüche, **dadurch gekennzeichnet, dass** es sich um:

- ein Copolymer, das sich von

  - A: SPE, vorzugsweise 5 bis 95 Mol-%, weiter bevorzugt 10 bis 90%, und
  - B: MAPTAC, vorzugsweise 5 bis 95 Mol-%,
  weiter bevorzugt 10 bis 90%,
  ableitet,

- ein Copolymer, das sich von

  - A: SPE, vorzugsweise 5 bis 95 Mol-%, weiter bevorzugt 10 bis 90 Mol-%, und
  - B: DIQUAT, vorzugsweise 5 bis 95 Mol-%,
  weiter bevorzugt 10 bis 90 Mol-%,
  ableitet,

- ein Copolymer, das sich von

  - A: SPE, vorzugsweise 5 bis 90 Mol-%, weiter bevorzugt 10 bis 80 Mol-%,
  - B: MAPTAC, vorzugsweise 5 bis 90 Mol-%, weiter bevorzugt 10 bis 80 Mol-%, und
  - C: Acrylamid, vorzugsweise 5 bis 90 Mol-%,
  weiter bevorzugt 10 bis 80 Mol-%,
  ableitet,

- ein Copolymer, das sich von

  - A: SPE, vorzugsweise 5 bis 90 Mol-%, weiter bevorzugt 10 bis 80 Mol-%,
  - B: DIQUAT, vorzugsweise 5 bis 90 Mol-%, weiter bevorzugt 10 bis 80 Mol-%, und
  - C: Acrylamid, vorzugsweise 5 bis 90 Mol-%,
  weiter bevorzugt 10 bis 80 Mol-%,
  ableitet,

- ein Copolymer, das sich von

  - A: SPP, vorzugsweise 5 bis 95 Mol-%, weiter bevorzugt 10 bis 90 Mol-%, und
  - B: MAPTAC, vorzugsweise 5 bis 95 Mol-%,
  weiter bevorzugt 10 bis 90 Mol-%,
  ableitet,

- ein Copolymer, das sich von

  - A: SPP, vorzugsweise 5 bis 95 Mol-%, weiter bevorzugt 10 bis 90 Mol-%, und
  - B: DIQUAT, vorzugsweise 5 bis 95 Mol-%, weiter bevorzugt 10 bis 90 Mol-%,
  ableitet,

- ein Copolymer, das sich von

  - A: SPP, vorzugsweise 5 bis 90 Mol-%, weiter bevorzugt 10 bis 80 Mol-%,
  - B: MAPTAC, vorzugsweise 5 bis 90 Mol-%, weiter bevorzugt 10 bis 80 Mol-%, und
  - C: Acrylamid, vorzugsweise 5 bis 90 Mol-%,

weiter bevorzugt 10 bis 80 Mol-%,
ableitet, oder

- ein Copolymer, das sich von

    - A: SPP, vorzugsweise 5 bis 90 Mol-%, weiter bevorzugt 10 bis 80 Mol-%,
    - B: DIQUAT, vorzugsweise 5 bis 90 Mol-%, weiter bevorzugt 10 bis 80 Mol-%, und
    - C: Acrylamid, vorzugsweise 5 bis 90 Mol-%, weiter bevorzugt 10 bis 80 Mol-%,

    ableitet,
    handelt.

**14.** Copolymer nach einem der vorhergehenden Ansprüche, **dadurch gekennzeichnet, dass** es wasserlöslich oder wasserdispergierbar ist.

**15.** Zusammensetzung zur Behandlung oder Modifizierung von Oberflächen, umfassend:

- einen Träger, der vorzugsweise flüssig ist,
- das Copolymer nach einem der vorhergehenden Ansprüche,
- gegebenenfalls ein Tensid,
- gegebenenfalls ein Salz, eine Säure und/oder eine Base und
- gegebenenfalls ein Mittel zur Behandlung oder Modifizierung der Oberfläche.

**16.** Zusammensetzung nach Anspruch 15, **dadurch gekennzeichnet, dass** sie ein anionisches oder amphoteres Tensid umfasst.

**17.** Zusammensetzung nach Anspruch 15, **dadurch gekennzeichnet, dass** sie ein nichtionisches Tensid umfasst.

**18.** Zusammensetzung nach einem der Ansprüche 15 bis 17, **dadurch gekennzeichnet, dass** die Einheiten B bei dem pH-Wert der Zusammensetzuing in kationischer Form vorliegen.

**19.** Zusammensetzung nach einem der Ansprüche 15 bis 19, **dadurch gekennzeichnet, dass**:

- sie ein anionisches oder amphoteres Tensid umfasst und
- sie Koazervate umfasst oder durch Verdünnen und/oder Modifizieren des pH-Werts Koazervate bildet.

**20.** Zusammensetzung nach Anspruch 19, **dadurch gekennzeichnet, dass** das Copolymer, das anionische oder amphotere Tensid, der Träger, gegebenenfalls andere Tenside, ein Salz, eine Base und/oder eine Säure und ihre Mengen so beschaffen sind, dass mindestens ein Teil des aus dem Copolymer, dem anionischen oder amphoteren Tensid, dem Träger und gegebenenfalls den anderen Tensiden, Salz, Base und/oder Säure bestehenden Sytems

- durch Verdünnen und/oder Modifizieren des pH-Werts Koazervate bildet oder
- Koazervate umfasst.

**21.** Zusammensetzung nach einem der Ansprüche 15 bis 20, **dadurch gekennzeichnet, dass** sie ein Mittel zur Behandlung oder Modifizierung der Oberfläche umfasst, wobei es sich bei dem Mittel um ein Polyorganosiloxan, ein Antischuppenmittel, einen Duftstoff, ein Öl oder ein UV-Schutzmittel handelt.

**22.** Zusammensetzung nach einem der Ansprüche 15 bis 21, **dadurch gekennzeichnet, dass** es sich um:

- eine kosmetische Zusammensetzung, die vorzugsweise zum Ausspülen bestimmt ist, vorzugsweise ein Shampoo, ein Produkt zum Frisieren der Haare, eine Haarpflegespülung, ein Haarpflegeprodukt, ein Hautpflegeprodukt oder ein Duschgel oder
- eine Reinigungszusammensetzung für die Haushalts-, Industrie- oder Institutionspflege, vorzugsweise zur Wäschepflege oder zur Reinigung oder Behandlung von harten Oberflächen, handelt.

**23.** Verfahren zur Reinigung oder Behandlung von harten Oberflächen, bei dem man:

- auf die Oberfläche eine Zusammensetzung nach einem der Ansprüche 15 bis 22 aufbringt und

- gegebenenfalls den Träger entfernt oder die Zusammensetzung verdünnt oder den pH-Wert modifiziert.

**24.** Verfahren nach Anspruch 23, **dadurch gekennzeichnet, dass** es sich bei der Oberfläche um die Haut und/oder die Haare handelt, wobei es sich bei der Behandlung oder Modifizierung um eine Konditionierung der Haut und/oder der Haare handelt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4075131 A
- US 6403073 B
- FR 2742657
- US 4534892 A
- EP 112592 A
- FR 2519863
- US 4994088 A
- US 4607076 A
- EP 532967 A
- WO 2004083354 A
- WO 0001746 A
- US 5026490 A
- US 6346588 B
- JP 11349826 A
- US 4565647 A
- EP 219048 A
- US 3959230 A
- US 3893929 A
- US 4116896 A
- US 4702857 A
- US 4770666 A
- US 4968451 A
- EP 540374 A
- FR 2728915 A
- FR 2236926 A
- US 4711730 A
- US 4721580 A
- US 4877896 A
- FR 2334698 A
- US 4597898 A
- EP 11984 A
- EP 76768CEE A
- WO 9216187 A
- US 3308067 A
- EP 66915 A
- EP 488868 A
- EP 561656 A
- US 4728455 A
- US 5114606 A
- US 5280117 A
- EP 909809 A
- US 5559261 A
- WO 9623859 A
- WO 9623860 A
- WO 9623861 A
- US 5108660 A
- US 5536452 A
- US 5587022 A


### Littérature non-brevet citée dans la description


- **I.V. Berlinova ; I.V. Dimitrov ; R.G. Kalinova ; N.G. Vladimirov.** Synthesis and aqueous solution behaviour of copolymers containing sulfobetaine moieties in side chains. *Polymer,* 2000, vol. 41, 831-837
- **Wen-Fu Lee ; Chun-Hsiung Lee.** Poly(sulfobetaine)s and corresponding cationic polymers: 3. Synthesis and dilute aqueous solution properties of poly(sulfobetaine)s derived from styrene-maleic anhydride. *Polymer,* 1997, vol. 38 (4), 971-979
- **Lee, Wen-Fu ; Chen, Yan-Ming.** Poly(sulfobetaine)s and corresponding cationic polymers. VIII. Synthesis and aqueous solution properties of a cationic poly(methyl iodide quaternized styrene-N,N-dimethylaminopropyl maleamidic acide) copolymer. *Journal of Applied Polymer Science,* 2001, vol. 80, 1619-1626
- **Andrew B. Lowe ; Norman C. Billingham ; Steven P. Armes.** Synthesis of polybetaines with narrow molecular mass distribution and controlled architecture. *Chem. Commun.,* 1996, 1555-1556
- **Andrew B. Lowe ; Norman C. Billingham ; Steven P. Armes.** Synthesis and Properties of Low- Polydispersity Poly(sulfopropylbetaine)s and Their Block Copolymers. *Macromolecules,* 1999, vol. 32, 2141-2146
- **T. Hamaide.** New polymeric phosphonato-, phosphinato- and carboxybétaïnes. *Makromolecular Chemistry,* 1986, vol. 187, 1097-1107
- **G. Robinson ; S.B. Ross Murphy ; E.R. Morris.** Viscosity-Molecular weight relationship, intrinsic chain flexibility and dynamic solution properties of guar galactomannan. *Carbohydrate Research,* 1982, vol. 107, 17-32